# EUROPEAN PATENT APPLICATION

(11) **EP 1 844 734 A2**
(43) Date of publication of application: **17.10.2007**
(21) Application number: 07015123.8
(22) Date of filing: 07.07.2005
(51) Int. Cl.: A61F 2/00

(54) **Combination drug therapy for reducing scar tissue formation**

(30) Priority: 08.07.2004 US 887272
(62) Divisional of application: 05770337.3
(71) Applicant: Afmedica, Inc., Kalamazoo, MI 49007 (US)
(72) Inventor: Shebuski, Roland J., Bergland, MI 49010 (US); Luderer, Jack R., Kalamazoo, MI 49024 (US); Fischell, Tim A., Kalamazoo, MI 49008 (US)
(74) Representative: Westphal, Thomas

(57) **Abstract**

The present invention describes various devices and methods wherein a cytostatic antiproliferative drug, either alone or in combination with other drugs, is placed between internal body tissues to prevent the formation of scar tissue and/or adhesions during healing of a wound or surgical site. Specific devices to achieve this administration include, but are not limited to, a permanent implant or a biodegradable material having an attached antiproliferative drug such as sirolimus. These antiproliferative drugs may be combined with other drugs including, but not limited to, antiplatelets, antithrombotics or anticoagulants. The present invention also contemplates methods to a reduce scar tissue and/or adhesions or adhesion formation at an anastomosis site. In particular, a cytostatic antiproliferative drug is administered to an arteriovenous shunt anastomoses in patients having end-stage renal disease.

## Description

### Field Of The Invention

The present invention relates to devices and methods to prevent the formation of scar tissue and/or adhesions following a surgical procedure, trauma or wound. In one embodiment, the present invention relates to medical devices comprising antiproliferative drugs. In another embodiment, the present invention related to devices and methods comprising antiplatelet drugs *(i.e.,* for example, a GPIIb/IIIa inhibitor). In another embodiment, the present invention relates to medical devices that prevent scar tissue and/or adhesion formation comprising a cytostatic antiproliferative drug in combination with other drugs including, but not limited to, antiplatelet drugs, antithrombotic drugs or anticoagulant drugs.

### Background

Post-operative scar tissue and/or adhesion formation and blood vessel narrowing are major problems following abdominal, neurological, vascular or other types of surgery. For example, narrowing of a blood vessel at the site of an anastomosis is often caused by the unwanted proliferation of scar tissue and/or adhesions at that location.

Excess post-operative scar tissue and/or adhesion formation and blood vessel narrowing are major problems following abdominal, neurological, spinal, vascular, thoracic or other types of surgery using both classical open and arthroscopic/laparoscopic procedures.

Scar tissue and/or adhesions forms as part of the natural healing process of an injury whereupon the body usually initiates a full and swift wound healing response resulting in reconstructed, repaired tissue. In certain instances, however, this normal healing process may result in excessive scar tissue and/or adhesions.

Following some kinds of surgery or injury, excess scar tissue and/or adhesions production is a major problem which influences the result of surgery and healing. In glaucoma surgery, for example, several anti-scarring and/or adhesion regimens are currently used to improve surgery results, but are of limited use clinically because of severe complications. Other examples of excess scar tissue and/or adhesions production negatively impacting the outcome of surgery include adhesion lysis surgery, angioplasty, spinal surgery, vascular surgery and heart surgery.

The current state of the art is lacking in post-surgical and post-trauma treatments to significantly reduce the formation of scar tissue and/or adhesions using drugs having a low medical risk and a high therapeutic benefit.

### Summary Of The Invention

The present invention relates to devices and methods to prevent the formation of scar tissue and/or adhesions following a surgical procedure, trauma or wound. In one embodiment, the present invention relates to medical devices comprising antiproliferative drugs. In another embodiment, the present invention related to devices and methods comprising antiplatelet drugs (*i*.*e*., for example, a GPIIb/IIIa inhibitor). In another embodiment, the present invention relates to medical devices that prevent scar tissue and/or adhesion formation comprising a cytostatic antiproliferative drug in combination with other drugs including, but not limited to, antiplatelet drugs, antithrombotic drugs or anticoagulant drugs.

The present invention is not limited to compositions and methods comprising a GPIIb/IIIa inhibitor and an antiproliferative. The present invention also contemplates embodiments analgous to all those described herein such that a GPIIb/IIIa inhibitor might be the only pharmaceutically active compound. In one embodiment, the present invention contemplates a composition attached to a medical device, said composition comprising a GPIIb/IIIa inhibitor. In another embodiment, the present invention contemplates a method of inhibiting or reducing fibrin sheath formation, scar tissue and/or adhesion formation comprising administering a GPIIb/IIIa inhibitor to a patient undergoing or following a surgical procedure resulting in, or at risk for developing, fibrin sheath formation, scar tissue and/or adhesion formation.

One embodiment of the present invention contemplates a composition attached to a polymeric medical device, said composition comprising a GPIIb/IIIa inhibitor. In one embodiment, the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the composition further comprises an antithrombin. In another embodiment, the composition further comprises an anticoagulant. In yet another embodiment, the composition provides a controlled release drug elution. In one embodiment, the composition comprises a hydrophyllic polymer that is covalently attached to said polymeric medical device. In one embodiment, said polymeric medical device comprises a polymer selected from the group including, but not limited to, silicone, polyurethane and polyvinylchloride. In one embodiment, the medical device is selected from the group comprising a dialysis/apheresis catheter, a dialysis catheter, a peritoneal dialysis catheter, a fixed-tip dialysis catheter. In another embodiment, the medical device comprises a synthetic vascular graft. In yet another embodiment, the medical device comprises an anti-adhesion membrane barrier. In one embodiment, the membrane barrier comprises oxidized regenerated cellulose.

One embodiment of the present invention contemplates a composition comprising a GPIIb/IIIa inhibitor. In one embodiment, embodiment the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the composition further comprises an antithrombin drug. In one embodiment, the antithrombin drug comprises bivalirudin. In one embodiment, the composition further comprises an anticoagulant drug. In one embodiment, the composition further comprises a polymer-based medium. In one embodiment, the medium provides a controlled release drug elution. In one embodiment, the polymer of said medium is selected from the group comprising polyvinyl pyrrolidone, poly(acrylic acid), poly(vinyl acetamide), poly(propylene glycol), poly(ethylene co-vinyl acetate), poly(n-butyl methacrylate), and poly(styrene-b-isobutylene-b-styrene). In one embodiment, the medium is attached to a medical device. In one embodiment, the medical device is selected from the group comprising a dialysis/apheresis catheter, a dialysis catheter, a peritoneal dialysis catheter, a fixed-tip dialysis catheter.

One embodiment of the present invention contemplates a composition comprising a PEA polymer, wherein said polymer comprises a GPIIb/IIIa inhibitor. In one embodiment, the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the composition further comprises an antithrombin drug. In one embodiment, the antithrombin drug comprises bivalirudin. In one embodiment, the composition further comprises an anticoagulant drug. In one embodiment, the polymer comprises lysine. In another embodiment, the polymer comprises leucine. In another embodiment, the polymer provides a controlled release drug elution. In one embodiment, the polymer is attached to a vascular wrap. In one embodiment, the polymer is attached to a medical device. In one embodiment, the medical device is selected from the group comprising a dialysis/apheresis catheter, a dialysis catheter, a peritoneal dialysis catheter, a fixed-tip dialysis catheter.

Another embodiment of the present invention contemplates a method, comprising: a) providing; i) a patient undergoing or following a surgical procedure, said procedure resulting in scar tissue and/or adhesion formation, ii) a composition comprising a GPIIb/IIIa inhibitor; and b) administering said composition to said patient under conditions such that said scar tissue and/or adhesion formation is reduced. In one embodiment, the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the surgical procedure is selected from the group comprising a kidney transplant and an anastomosis. In one embodiment, the administering comprises a membrane barrier. In another embodiment, the administering comprises an aqueous solution, wherein said solution polymerizes upon contact with said patient.

Another embodiment of the present invention contemplates a method, comprising: a) providing; i) a patient undergoing or following a surgical procedure, said procedure resulting in scar tissue and/or adhesion formation, ii) a composition comprising a PEA polymer, wherein said polymer comprises a GPIIb/IIIa inhibitor; and b) administering said composition to said patient under conditions such that said scar tissue and/or adhesion formation is reduced. In one embodiment, the polymer further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the surgical procedure is selected from the group comprising a kidney transplant and an anastomosis. In one embodiment, the composition further comprises an antithrombin drug. In one embodiment, the antithrombin drug comprises bivalirudin. In one embodiment, the composition further comprises an anticoagulant drug. In one embodiment, the polymer comprises lysine. In another embodiment, the polymer comprises leucine. In another embodiment, the polymer provides a controlled release drug elution. In one embodiment, the polymer is attached to a vascular wrap. In one embodiment, the polymer is attached to a medical device. In one embodiment, the medical device is selected from the group comprising a dialysis/apheresis catheter, a dialysis catheter, a peritoneal dialysis catheter, a fixed-tip dialysis catheter.

Another embodiment of the present invention contemplates a method, comprising: a) providing; i) a patient undergoing or following a surgical procedure, said procedure having a risk of scar tissue and/or adhesion formation, ii) a composition comprising a GPIIb/IIIa inhibitor; and b) administering said composition to said patient under conditions such that said scar tissue and/or adhesion formation is reduced. In one embodiment, the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the surgical procedure is selected from the group comprising a kidney transplant and an anastomosis. In one embodiment, the administering comprises a membrane barrier. In another embodiment, the administering comprises an aqueous solution, wherein said solution polymerizes upon contact with said patient.

Another embodiment of the present invention contemplates a method, comprising: a) providing; i) a patient undergoing or following a surgical procedure, said procedure having a risk of scar tissue and/or adhesion formation, ii) a composition comprising a PEA polymer, wherein said polymer comprises a GPIIb/IIIa inhibitor; and b) administering said composition to said patient under conditions such that said scar tissue and/or adhesion formation is reduced. In one embodiment, the polymer further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the surgical procedure is selected from the group comprising a kidney transplant and an anastomosis. In one embodiment, the composition further comprises an antithrombin drug. In one embodiment, the antithrombin drug comprises bivalirudin. In one embodiment, the composition further comprises an anticoagulant drug. In one embodiment, the polymer comprises lysine. In another embodiment, the polymer comprises leucine. In another embodiment, the polymer provides a controlled release drug elution. In one embodiment, the polymer is attached to a vascular wrap. In one embodiment, the polymer is attached to a medical device. In one embodiment, the medical device is selected from the group comprising a dialysis/apheresis catheter, a dialysis catheter, a peritoneal dialysis catheter, a fixed-tip dialysis catheter.

Another embodiment of the present invention contemplates a method, comprising a) providing; i) a patient undergoing a dialysis catheter placement procedure, said procedure resulting in fibrin sheath formation; ii) a composition attached to a dialysis catheter, said composition comprising a GPIIb/IIIa inhibitor wherein said catheter is placed in said patient to perform said dialysis procedure; and b) placing said catheter in said patient under conditions such that fibrin sheath formation is reduced. In one embodiment, the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the catheter is selected from the group comprising a peritoneal catheter and a femoral catheter. In one embodiment, the catheter comprises an non-adhesive luminal surface.

Another embodiment of the present invention contemplates a method, comprising a) providing; i) a patient undergoing a dialysis catheter placement procedure, said procedure having a risk of fibrin sheath formation; ii) a composition attached to a dialysis catheter, said composition comprising a GPIIb/IIIa inhibitor wherein said catheter is placed in said patient to perform said dialysis procedure; and b) placing said catheter in said patient under conditions such that fibrin sheath formation is reduced. In one embodiment, the composition further comprises rapamycin. In one embodiment, the GPIIb/IIIa inhibitor is selected from the group comprising xemilofiban, cromafiban, elarofiban, orbofiban, roxifiban, sibrafiban, RPR 109891, UR-4033, UR-3216, UR-2922, abciximab, tirofiban, or eptifibatide. In one embodiment, the catheter is selected from the group comprising a peritoneal catheter and a femoral catheter. In one embodiment, the catheter comprises an non-adhesive luminal surface.

Another embodiment of the present invention contemplates a composition for a hydrogel-based bioadhesive comprising: i) a first medium comprising sirolimus and analogs of sirolimus and a functional polymer and ii) a second medium comprising a small crosslinker molecule. In one embodiment, the crosslinker molecule is selected from the group comprising ethoxylated glycerols, inositols, trimethylolpropanes, succinates, glutarates, glycolate/2-hydroxybutyrate and glycolate/4-hydroxyproline. In another embodiment, the functional polymer is selected from the group comprising polyethylene oxide and polyethylene glycol. In one embodiment, the first medium further comprises a supplemental or complementary drug selected from the group comprising an antiplatelet drug, an antithrombin drug, an anticoagulant drug or an antiinflammatory drug.

Another embodiment of the present invention contemplates a method for contacting a surgical site with a hydrogel-based bioadhesive comprising: a) providing; i) a surgical site; and ii) a syringe comprising; I) a first barrel containing a first aqueous medium comprising sirolimus and analogs of sirolimus and a functional polymer; and II) a second barrel containing a second aqueous medium comprising a small crosslinker molecule; b) contacting said first and second mediums with said surgical site under conditions such that said first and second mediums are mixed; c) crosslinking said mixed first and second mediums initiated by a self-polymerizing reaction to create a bioadhesive layer. In one embodiment, the contacting comprises spraying. In one embodiment, the contacting of the first and second mediums comprises a sequential order. In one embodiment, the first and second mediums are mixed prior to contacting the surgical site. In one embodiment, the first aqueous medium further comprises a supplemental or complementary drug selected from the group comprising an antiplatelet drug, an antithrombin drug, an anticoagulant drug or an antiinflammatory drug.

The present invention also relates to devices and methods comprising sirolimus, tacrolimus and analogs of sirolimus to achieve reductions in scar tissue and/or adhesion formation. In one embodiment, the formation of scar tissue and/or adhesions is reduced following a surgical procedure. In one embodiment, the present invention relates to surgical wraps comprising a GPIIIa/IIb inhibitor that reduce scar tissue and/or adhesion formation following a surgical procedure. In one embodiment, the wrap further comprises sirolimus, tacrolimus and analogs of sirolimus. In another embodiment, the present invention relates to surgical wraps that reduce scar tissue and/or adhesion formation comprising a GPIIIa/IIb inhibitor and/or a cytostatic antiproliferative drug (*i.e*., for example, sirolimus, tacrolimus and analogs of sirolimus) following a surgical procedure in a patient having end stage renal disease. In another embodiment, the present invention further comprises an antiplatelet or an antithrombin drug. In another embodiment, the present invention further comprises an anticoagulant drug.

Several embodiments of this invention comprise methods for prophylactic treatment of vasculoproliferative disease following construction of an arteriovenous graft, an arterial-arterial graft, or an arteriovenous fistula. Other embodiments comprise treatments for established vasculoproliferative disease following construction of an arteriovenous graft, an arterial-arterial graft, or an arteriovenous fistula. Other embodiments comprise treatments for the reduction and/or prevention of fibrin sheath formation. Although it is not necessary to understand the mechanism of an invention, it is believed that treatments described herein related to vasculoproliferative disease may involve thrombosis, thromboembolism and thrombic occlusion.

One embodiment of the present invention contemplates a device comprising a cytostatic anti-proliferative drug attached to a surgical material designed to be placed generally around (*i.e.*, for example, next to) patient tissue that has been surgically joined or surgically treated. In one embodiment, said cytostatic anti-proliferative drug prevents the formation of excess post-operative scar tissue and/or adhesions *(i.e.,* results in an overall reduction in scar tissue and/or adhesion formation). In one embodiment, the surgical material comprises a suture. In another embodiment, the surgical material comprises a mesh or gauze *(i.e.,* for example, a woven or knitted solid sheet). In one embodiment, the mesh or gauze comprises fibers. In one embodiment, the surgical material comprises interstices *(i.e.,* for example, air holes). In one embodiment, the surgical material comprises a sponge. In another embodiment, the surgical material comprises a staple. In another embodiment, the surgical material to which the drug is attached may be either a permanent implant or it may be biodegradable. In one embodiment, the drug may be attached to an absorbable hemostat gauze *(i.e.,* for example Surgicel^{™}, Johnson & Johnson) or a Vicryl mesh product. In one embodiment, the cytostatic anti-proliferative drug comprises sirolimus, tacrolimus or analogs of sirolimus. In another embodiment, the surgical material further comprises an antiplatelet or an antithrombin drug. In another embodiment, the surgical material further comprises an anticoagulant drug. In one embodiment, the drug is released from a biodegradable surgical material, decreases cellular proliferation and reduces the formation of and/or adhesion at or near a surgical site. In another embodiment, the drug is released from a biodegradable surgical material, decreases cellular proliferation and reduces the formation of thrombosis at, or near, a surgical site. In one embodiment, the method of using the device further comprises a systemic administration of a complementary pharmaceutical drug. In one embodiment, the systemic administration may be selected from the group including, but not limited to, oral ingestion, by a transdermal patch, by a cream or ointment applied to the skin, by inhalation and by a suppository. In one embodiment, the complementary pharmaceutical drug includes, but not limited to, a cytostatic antiproliferative (*i*.*e*., for example, sirolimus, tacrolimus, or analogs of sirolimus), antiinflammatory drugs, cortiocosteroids, antithrombotics, antiplatelets, antibiotics, antibacterials, antivirals, analgesics, and anesthetics. In one embodiment, the complementary pharmaceutical drug is administered starting from between at least one hour to as long as 5 days prior to a surgical procedure. In another embodiment, the complementary pharmaceutical drug is administered for a period of between at least one day to as long as sixty (60) days after the procedure. It should be understood that the complementary pharmaceutical drug could be given systemically without using any of the devices described herein. It should be understood that the complementary pharmaceutical drug could be given systemically in addition to the application of the cytostatic anti-proliferative drug attached to any one or more of the devices described herein. It should also be understood that an optimum result might be obtained with using one cytostatic anti-proliferative drug attached to a device with a plurality of different complementary pharmaceutical drugs being used for systemic administration. It is known to those skilled in the art that the dose of the complementary pharmaceutical drug depends on the specific drug used, the patient's condition *(i.e.,* general state of health and well being) and characteristics *(i.e.,* for example, body weight, height, age, metabolism, pre-existing conditions *etc.).*

Another embodiment of the present invention contemplates a surgical closure material comprising a GPIIb/IIIa inhibitor. In one embodiment, the surgical closure material further comprises an antiplatelet or an antithrombin drug. In another embodiment, the surgical closure material further comprises an anticoagulant drug. In one embodiment, the closure comprises a suture. In another embodiment; the closure comprises a staple. In one embodiment, the closure is used to join body tissues. In one embodiment, the closure is used to join two blood vessels. In one embodiment, the blood vessel joining comprises an anastomosis. In one embodiment, the attached drug is released from the closure and causes a reduction of cellular proliferation and therefore scar tissue and/or adhesion formation at the site of suture penetration of the vessel wall. In one embodiment, the closure material is placed within the skin. In another embodiment, the closure material is used during a method of plastic surgery. In another embodiment, the closure material is used during eye surgery. In one embodiment, the closures are bioresorbable. In another embodiment, the closures are non-bioresorbable.

Another embodiment of the present invention contemplates a surgical material comprising a cytostatic anti-proliferative drug capable of being placed into or wrapped generally around a surgical procedure site wherein said drug reduces scar tissue and/or adhesion formation at the site of the surgical procedure. In another embodiment, the surgical material further comprises an antiplatelet or an antithrombin drug. In another embodiment, the surgical material further comprises an anticoagulant drug. In one embodiment, the surgical material is wrapped around the surgical procedure site selected from the group including, but not limited to, a blood vessel, a ureter, a bile duct, a fallopian tube, and any other vessel of the human body at the site of a surgically created anastomosis. In one embodiment, the drug is released from the wrap and reduces scar tissue and/or adhesion formation at, or near, the anastomosis site.

Another embodiment of the present invention contemplates a biodegradable surgical material or mesh suitable for placement between body tissues comprising an attached drug that elutes slowly from the surgical material to reduce cellular proliferation associated with post-surgical adhesions and/or scar tissue and/or adhesion formation. In another embodiment, the surgical material further comprises an antiplatelet or an antithrombin drug. In another embodiment, the surgical material further comprises an anticoagulant drug. In one embodiment, the attached drug comprises a cytostatic anti-proliferative drug such as, sirolimus, tacrolimus or analogs of sirolimus.

Another embodiment of the present invention contemplates a device capable of placement into the body of a patient, wherein the device has an attached cytostatic anti-proliferative drug. In another embodiment, the device further comprises an antiplatelet or an antithrombin drug. In another embodiment, the device further comprises an anticoagulant drug. In one embodiment, the placement of the device further comprises administering a complementary pharmaceutical drug. In one embodiment, the device comprises a mesh, gauze or bandage. In another embodiment, the device comprises a medical device. In one embodiment, the complementary pharmaceutical drug may be the same or different cytostatic anti-proliferative drug administered as a systemic medication from some time prior to a surgical procedure and/or for some time after that procedure in order to reduce excessive post-surgical scar tissue and/or adhesion formation. In one embodiment, the patient is a human. In another embodiment, the patient is a non-human animal.

One embodiment of the present invention contemplates a surgical material comprising a surgical wrap and a GPIIb/IIIa inhibitor. In one embodiment, the wrap further comprises a cytostatic antiproliferative drug. In one embodiment, the cytostatic antiproliferative drug is attached to a medium. In another embodiment, the surgical material further comprises an antiplatelet or an antithrombin drug. In another embodiment, the surgical material further comprises an anticoagulant drug. In one embodiment, the surgical wrap is completely covered by the medium. In another embodiment, the surgical wrap is partially covered by the medium. In another embodiment, the cytostatic antiproliferative drug is attached to the surgical wrap. In one embodiment, the medium comprises at least one cytostatic antiproliferative drug selected from the group including, but not limited to, sirolimus, anti-sense to c-myc, tacrolimus, everolimus, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. In one embodiment, the surgical wrap comprises an annular wrap. In another embodiment, the surgical wrap comprises a slit annular wrap. In another embodiment, the surgical wrap comprises a flat rectangle. In another embodiment, the surgical wrap comprises a surgical closure. In one embodiment, the surgical closure is selected from the group including, but not limited to, a suture and a staple. In one embodiment, the surgical wrap is biodegradable. In one embodiment, the biodegradable surgical wrap comprises at least one poly-lactide polymer. In another embodiment, the biodegradable surgical wrap comprises at least one poly-glycolide polymer. In one embodiment, the surgical wrap is drug-eluting. In one embodiment, the surgical wrap is biostable. In one embodiment, the medium comprises microparticles, liposomes, gels, hydrogels, xerogels and foams. In another embodiment, the surgical wrap further comprises a supplemental pharmaceutical drug. In another embodiment, the surgical wrap further comprises at least one surgical closure, wherein said closure is capable of securing an anastomosis.

Another embodiment of the present invention contemplates a method, comprising: a) providing; i) a patient undergoing or following a surgical procedure, ii) at least one complementary pharmaceutical drug, and iii) a cytostatic antiproliferative drug; and b) administering said cytostatic antiproliferative drug in combination with said complementary pharmaceutical drug to said subject wherein the outcome of said surgical procedure is improved. In one embodiment, said complementary pharmaceutical drug is selected from the group including, but not limited to, cytostatic antiproliferative drugs, antiinflammatory drugs, corticosteroids, antithrombotics, antiplatelets, antibiotics, antibacterials, antivirals, antiseptics, analgesics and anesthetics. In another embodiment, the method further comprises an antiplatelet or an antithrombin drug. In another embodiment, the method further comprises an anticoagulant drug. In one embodiment, said cytostatic antiproliferative drug is selected from the group including, but not limited to, sirolimus, anti-sense to c-myc, tacrolimus, everolimus, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. In one embodiment, the method further comprises administering a second cytostatic antiproliferative drug in a skin ointment. In one embodiment, the method further comprises contacting a surgical material comprising a third cytostatic antiproliferative drug to a surgical site. In one embodiment, the surgical material comprises a flat rectangle. In another embodiment, the surgical material comprises a surgical closure. In one embodiment, the surgical closure is selected from the group including, but not limited to, a suture and a staple. In one embodiment, the surgical material comprises a surgical wrap. In one embodiment, the surgical material comprises an annular surgical wrap. In one embodiment, the surgical material comprises a slit annular surgical wrap.

Another embodiment of the present invention contemplates a method, comprising: a) providing; i) a patient undergoing or following a surgical procedure, and ii) a surgical material comprising a cytostatic antiproliferative drug; and b) contacting said surgical material with tissues of said patient under conditions that the formation of scar tissue and/or adhesions is decreased. In one embodiment, the cytostatic antiproliferative drug is selected from the group including, but not limited to, sirolimus, anti-sense to c-myc, tacrolimus, everolimus, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. In one embodiment, the surgical material further comprises an antiplatelet or an antithrombin drug. In another embodiment, the surgical material further comprises an anticoagulant drug. In one embodiment, the surgical procedure comprises an anastomosis. In one embodiment, the anastomosis comprises vessels selected from the group including, but not limited to, an artery, a vein, a ureter, a urethra, an artificial graft, a jejunum, an ileum, a duodenum, a colon, a bile duct or a fallopian tube. In one embodiment, the method further comprises administering at least one complementary pharmaceutical drug at least one day prior to said surgical procedure. In one embodiment, the complementary pharmaceutical drug is selected from the group including, but not limited to, cytostatic antiproliferative drugs, antiinflammatory drugs, corticosteriods, antithrombotics, antiplatelets, antibiotics, antibacterials, antivirals, antiseptics, analgesics and anesthetics. In one embodiment, the method further comprises administering at least one second complementary pharmaceutical drug at least one day after said surgical procedure. In one embodiment, the second complementary pharmaceutical drug is selected from the group including, but not limited to, cytostatic antiproliferative drugs, antiinflammatory drugs, corticosteriods, antithrombotics, antiplatelets, antibiotics, antibacterials, antivirals, antiseptics, analgesics and anesthetics. In one embodiment, the anastomosis comprises a vein and an aorta. In one embodiment, the anastomosis comprises an internal mammary artery and a coronary artery.

Another embodiment of the present invention contemplates a method, comprising: a) providing, i) a patient having a wound, ii) a medium comprising a cytostatic antiproliferative drug, and iii) a bandage; b) contacting said medium with said wound; and c) placing said bandage over said medium under conditions such that scar tissue and/or adhesion formation is decreased. In one embodiment, the method further comprises at least one supplemental pharmaceutical drug, wherein said drug is attached to said medium. In one embodiment, the medium further comprises an antiplatelet or an antithrombin drug. In another embodiment, the medium further comprises an anticoagulant drug.

Another embodiment of the present invention contemplates a method, comprising: a) providing, i) a patient undergoing or following a surgical procedure; ii) a surgical material comprising a cytostatic antiproliferative drug, wherein said material is capable of eluting said drug for at least one day; and b) placing said surgical material at or near said surgical procedure under conditions such that the formation of scar tissue and/or adhesions is decreased. In one embodiment, the surgical material further comprises an antiplatelet or an antithrombin drug. In one embodiment, the surgical material further comprises an anticoagulant drug. In one embodiment, the cytostatic antiproliferative drug prevents the initiation of cellular DNA replication at or before the S-phase of cellular mitosis.

Another embodiment of the present invention contemplates a method, comprising: a) providing; i) a patient, wherein said patient has at least one symptom of a renal disease; and ii) a surgical material comprising a cytostatic antiproliferative drug, wherein said surgical material is configured for extravascular placement; b) placing said surgical material extravascularly (*i*.*e*., for example, on the surface of a renal artery). In one embodiment, the renal disease comprises atherosclerosis. In another embodiment, the renal disease comprises end-stage renal disease. In yet another embodiment, the renal disease comprises nephropathy. In one embodiment, the patient further comprises at least one symptom of vascular stenosis or vascular restenosis *(i.e.,* for example, the renal artery). In one embodiment, the method further comprises reducing the stenosis or restenosis. In one embodiment, the stenosis or restenosis reduction comprises a reduction in scar tissue and/or adhesion formation. In another embodiment, the stenosis or restenosis reduction comprises a reduction in adhesion formation. In one embodiment, the vascular stenosis or restenosis results from a vascular access site. In one embodiment, the vascular access site is selected from the group including, but not limited to, an arteriovenous fistula and an arteriovenous graft. In one embodiment, the vascular access site comprises an anastomosis. In one embodiment, the arteriovenous graft comprises polytetrafluoroethylene. In one embodiment, the patient is selected from the group including, but not limited to human adults and human children. In one embodiment, the patient comprises a non-human animal *(i.e.,* for example, a dog, cat, bird, horse, sheep *etc.).* In one embodiment, the cytostatic antiproliferative drug is selected from the group including, but not limited to, sirolimus, anti-sense to c-myc, tacrolimus, everolimus, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. In one embodiment, the surgical material further comprises an antiplatelet or an antithrombin drug. In another embodiment, the surgical material further comprises an anticoagulant drug. In one embodiment, the method further comprises administering a complementary pharmaceutical drug to the patient. In one embodiment, the method further comprises administering a supplementary pharmaceutical drug to the patient. In one embodiment, the cytostatic antiproliferative drug is attached to a medium. In one embodiment, the medium is selected from the group including, but not limited to, microparticles, liposomes, gels, hydrogels, xerogels, foams and bioadhesives. In one embodiment, the placing of the surgical material comprises an open surgical site. In another embodiment, the placing of the surgical material comprises a closed surgical site. In one embodiment, the surgical material is selected from the group including, but not limited to, surgical sleeves, surgical wraps, annular surgical wraps and slit annular surgical wraps. In one embodiment, the placing of said surgical material is secured with a surgical closure. In one embodiment, the placing of the surgical material comprises a catheter or an endoscope.

Another embodiment of the present invention contemplates a method, comprising: a) providing; i) a patient, wherein said patient has a transplanted kidney in communication with a renal artery, wherein said renal artery is at risk for stenosis or restenosis; and ii) a surgical material comprising a cytostatic antiproliferative drug configured for placement on the exterior surface *(i.e.,* for example, extravascularly) of said renal artery; b) placing said surgical material on the exterior surface of said renal artery under conditions such that the risk of stenosis or restenosis of said renal artery is reduced. In one embodiment, the cytostatic antiproliferative drug is selected from the group including, but not limited to, sirolimus, anti-sense to c-myc, tacrolimus, everolimus, CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. In one embodiment, the surgical material further comprises an antiplatelet or an antithrombin drug. In another embodiment, the surgical material further comprises an anticoagulant drug. In one embodiment, the cytostatic antiproliferative drug is attached to a medium. In one embodiment, the medium is selected from the group including, but not limited to, microparticles, liposomes, gels, hydrogels, xerogels, foams and bioadhesives. In one embodiment, the method further comprises administering a complementary pharmaceutical drug to the patient. In one embodiment, the method further comprises administering a supplementary pharmaceutical drug to the patient. In one embodiment, the surgical material is selected from the group including, but not limited to, surgical sleeves, surgical wraps, annular surgical wraps and slit annular surgical wraps. In one embodiment, the placing of said surgical material is secured with a surgical closure.

These and other embodiments of this invention will become obvious to a person of ordinary skill in this art upon reading of the detailed description of this invention including the associated drawings.

### Definitions

The term "attached" as used herein, refers to any interaction between a medium (or carrier) and a drug. Attachment may be reversible or irreversible. Such attachment includes, but is not limited to, covalent bonding, and non-covalent bonding including, but not limited to, ionic bonding, Van der Waals forces or friction, and the like. A drug is attached to a medium (or carrier) if it is impregnated, incorporated, coated, in suspension with, in solution with, mixed with, *etc*.

The term "covalent bonding" as used herein, refers to an attachment between two compounds (*i.e.*, for example, a medium and a drug) that comprising a sharing of electrons.

The term "placing" as used herein, refers to any physical relationship (*i*.*e*., secured or unsecured) between a patient's biological tissue and a surgical material, wherein the surgical material comprises a pharmaceutical drug that may be, optionally, attached to a medium. Such a physical relationship may be secured by methods such as, but not limited to, gluing, suturing, stapling, spraying, laying, impregnating, and the like.

The term "exterior surface" as used herein, refers to the outside surface of any organ, vessel or epithelial tissue layer. For example, a surgical material may be placed on the exterior surface of a renal artery *(i.e.,* for example, extravascularly), as opposed to within the vascular luminal interior space.

The term "wound" as used herein, denotes a bodily injury with disruption of the normal integrity of tissue structures. In one sense, the term is intended to encompass a "surgical site". In another sense, the term is intended to encompass wounds including, but not limited to, contused wounds, incised wounds, lacerated wounds, non-penetrating wounds (*i*.*e*., wounds in which there is no disruption of the skin but there is injury to underlying structures), open wounds, penetrating wound, perforating wounds, puncture wounds, septic wounds, subcutaneous wounds, burn injuries *etc*. Conditions related to wounds or sores which may be successfully treated according to the invention are skin diseases.

The term "surgical site" as used herein, refers to a site created by any opening in the skin or internal organs performed for a specific medical purpose. The surgical site may be "open" where medical personnel have direct physical access to the area of interest as in traditional surgery. Alternatively, the surgical site may be "closed" where medical personnel perform procedures using remote devices such as, but not limited to, catheters wherein fluoroscopes may be used to visualize the activities and; endoscopes (*i.e.*, laparoscopes) wherein fiber optic systems may be used to visualize the activities. A surgical site may include, but is not limited to, organs, muscles, tendons, ligaments, connective tissue and the like.

The term "organ" as used herein, include, without limitation, veins, arteries, lymphatic vessels, esophagus, stomach, duodenum, jejunum, ileum, colon, rectum, urinary bladder, ureters, gall bladder, bile ducts, pancreatic duct, pericardial sac, peritoneum, heart, eyes, ears and pleura.

The term "skin" is used herein, very broadly embraces the epidermal layer of the skin and, if exposed, also the underlying dermal layer. Since the skin is the most exposed part of the body, it is particularly susceptible to various kinds of injuries such as, but not limited to, ruptures, cuts, abrasions, bums and frostbites or injuries arising from the various diseases.

The term "vessel" as used herein, refers to any biological organ that is roughly cylindrical in shape and comprises a lumen. Such a vessel may be joined to another vessel by a surgical procedure comprising anastomosis. For example, vessels include, but are not limited to, blood vessels, gastrointestinal tract, biliary duct, fallopian tubes, lymphatic ducts, bronchial tubules, and the like.

The term "anastomosis" as used herein, refers to a surgical procedure where two vessels or organs, each having a lumen, are placed in such proximity that growth is stimulated and the two vessels or organs are joined by forming continuous tissue *(i.e.,* for example, vascular organs such as veins or arteries *etc*.). Alternatively, non-vasculature organs may also be joined by an anastomosis (*i.e.*, gastrointestinal tract organs, lymphatic vessels, gall bladder and bile duct organs, kidney tubules *etc*.). One of skill in the art will recognize that an anastomosis procedure contemplated by the present invention is not limited to vascular surgery but includes all surgical procedures that join organs. Examples of anastomoses that can be performed include, but are not limited to, arterial anastomosis, venous anastomosis, arterio-venous anastomosis, anastomosis of lymphatic vessels, gastroesophageal anastomosis, gastroduodenal anastomosis, gastrojejunal anastomosis, anastomosis between and among the jejunum, ileum, colon and rectum, ureterovesicular anastomosis, anastomosis of the gall bladder or bile duct to the duodenum, and anastomosis of the pancreatic duct to the duodenum. In addition, an anastomosis may join an artificial graft *(i.e.,* for example, a vascular graft) to a bodily organ that has a lumen *(i.e.,* for example, a blood vessel).

The term "communication" as used herein, refers to the ability of two organs to exchange body fluids by flowing or diffusing from one organ to another in the manner typically associated with the organ pair that has been joined. Examples of fluids that might flow through an anastomosis include, but are not limited to, liquid and semi-solids such as blood, urine, lymphatic fluid, bile, pancreatic fluid, ingesta and purulent discharge.

The term "medium" as used herein, refers to any material, or combination of materials, which serve as a carrier or vehicle for delivering of a drug to a treatment point (e.g., wound, surgical site *etc.).* For all practical purposes, therefore, the term "medium" is considered synonymous with the term "carrier". It should be recognized by those having skill in the art that a medium comprises a carrier, wherein said carrier is attached to a drug or drug and said medium facilitates delivery of said carrier to a treatment point. Further, a carrier may comprise an attached drug wherein said carrier facilitates delivery of said drug to a treatment point. Preferably, a medium is selected from the group including, but not limited to, foams, gels (including, but not limited to, hydrogels), xerogels, microparticles *(i.e.,* microspheres, liposomes, microcapsules *etc*.), bioadhesives, or liquids. Specifically contemplated by the present invention is a medium comprising combinations of microparticles with hydrogels, bioadhesives, foams or liquids. Preferably, hydrogels, bioadhesives and foams comprise any one, or a combination of, polymers contemplated herein. Any medium contemplated by this invention may comprise a controlled release formulation. For example, in some cases a medium constitutes a drug delivery system that provides a controlled and sustained release of drugs over a period of time lasting approximately from 1 day to 6 months.

The term "xerogel" as used herein, refers to any device comprising a combination of silicone and oxygen having a plurality of air bubbles and an entrapped drug. The resultant glassy matrix is capable of a controlled release of an entrapped drug during the dissolution of the matrix.

The term "reduction in scar tissue and/or adhesion formation" as used herein refers to any tissue response that reflects an improvement in wound healing. Specifically, improvement in conditions such as, but not limited to, hyperplasia or adverse reactions to post-cellular trauma are contemplated. It is not contemplated that all scar tissue and/or adhesions must be avoided. It is enough if the amount of scarring and/or adhesions or hyperplasia is reduced as compared to untreated patients (e.g., as noted in published reports, historical studies *etc.).*

The term "foam" as used herein, refers to a dispersion in which a large proportion of gas, by volume, is in the form of gas bubbles and dispersed within a liquid, solid or gel. The diameter of the bubbles are usually relatively larger than the thickness of the lamellae between the bubbles.

The term "gel" as used herein, refers to any material forming, to various degrees, a medium viscosity liquid or a jelly-like product when suspended in a solvent. A gel may also encompass a solid or semisolid colloid containing a certain amount of water. These colloid solutions are often referred to in the art as hydrosols. One specific type of gel is a hydrogel. The term "hydrogel" as used herein, refers to any material forming, to various degrees, a jelly-like product when suspended in a solvent, typically water or polar solvents comprising such as, but not limited to, gelatin and pectin and fractions and derivatives thereof. Typically, a hydrogel is capable of swelling in water and retains a significant portion of water within its structure without dissolution. In one embodiment, the present invention contemplates a gel that is liquid at lower than body temperature and forms a firm gel when at body temperature.

The term "drug" or "compound" as used herein, refers to any pharmacologically active substance capable of being administered which achieves a desired effect. Drugs or compounds can be synthetic or naturally occuring, non-peptide, proteins or peptides, oligonucleotides or nucleotides, polysaccharides or sugars. Drugs or compounds may have any of a variety of activities, which may be stimulatory or inhibitory, such as antibiotic activity, antiviral activity, antifungal activity, steroidal activity, cytotoxic, cytostatic, anti-proliferative, anti-inflammatory, analgesic or anesthetic activity, or can be useful as contrast or other diagnostic agents. Drugs or compounds are capable of reducing wound or post-surgical scarring and/or adhesions (*i.e.*, for example, the activity of a drug or compound may be cytostatic). Although it is not necessary to understand the mechanism of an invention it is believed that one specific cytostatic drug might act by interrupting the cell division cycle in the G0 or G1 stage after binding to the Mammalian Target Of Rapamycin *(i.e.,* mTOR) protein; thus inhibiting proliferation without killing the cell. It is not intended that the term drug or compound refers to any non-pharmaceutically active material such as, but not limited to, polymers or resins intended for the creation of any one specific medium. Any drug or compound contemplated herein may be administered having an "effective dose".

The term "effective dose" as used herein refers to the concentration of any compound or drug contemplated herein that results in a favorable clinical response. An effective dose may range between approximately 1 ng/cm² - 100 mg/cm², preferably between 100 ng/cm² - 10 mg/cm², but more preferably between 500 ng/cm² - 1 mg/cm².

The term "rapamycin" as used herein refers to a compound represented by the drug sirolimus. Rapamycin is a macrocyclic lactone which may be naturally produced and isolated from a streptomycetes, *e.g., Streptomycetes hygroscopicus,* chemically synthesized or produced by genetic engineering cell culture techniques.

The term "analog" as used herein, refers to any compound having substantial structure-activity relationships to a parent compound such that the analog has similar biochemical activity as the parent compound. For example, sirolimus has many analogs that are substituted at either the 2-, 7- or 32- positions. One of skill in the art should understand that the term "derivative" is used herein interchangeably with term "analog".

The term "administered" or "administering" a drug or compound, as used herein, refers to any method of providing a drug or compound to a patient such that the drug or compound has its intended effect on the patient. For example, one method of administering is by an indirect mechanism using a medical device such as, but not limited to a catheter, applicator gun, syringe *etc*. A second exemplary method of administering is by a direct mechanism such as, local tissue administration *(i.e.,* for example, extravascular placement), oral ingestion, transdermal patch, topical, inhalation, suppository *etc*.

The term "extravascular placement", as used herein, refers to placing any device or composition at, or near, the periadvential region of a blood vessel.

The term "biocompatible", as used herein, refers to any material does not elicit a substantial detrimental response in the host. There is always concern, when a foreign object is introduced into a living body, that the object will induce an immune reaction, such as an inflammatory response that will have negative effects on the host. In the context of this invention, biocompatibility is evaluated according to the application for which it was designed: for example; a bandage is regarded a biocompatible with the skin, whereas an implanted medical device is regarded as biocompatible with the internal tissues of the body. Preferably, biocompatible materials include, but are not limited to, biodegradable and biostable materials.

The term "biodegradable" as used herein, refers to any material that can be acted upon biochemically by living cells or organisms, or processes thereof, including water, and broken down into lower molecular weight products such that the molecular structure has been altered.

The term "bioerodible" as used herein, refers to any material that is mechanically worn away from a surface to which it is attached without generating any long term inflammatory effects such that the molecular structure has not been altered. In one sense, bioerosion represents the final stages of "biodegradation" wherein stable low molecular weight products undergo a final dissolution.

The term "bioresorbable" as used herein, refers to any material that is assimilated into or across bodily tissues. The bioresorption process may utilize both biodegradation and/or bioerosion.

The term "biostable" as used herein, refers to any material that remains intact within a physiological environment for an intended duration resulting in a medically beneficial effect.

The term "supplemental pharmaceutical drug" as used herein, refers to any drug administered as part of a medium as contemplated by this invention. Administration of a medium comprising a supplemental phannaceutical drug includes, but is not limited to, systemic, local delivery, implantation, or any other means. A supplemental pharmaceutical drug may have activities similar to, or different from a drug capable being cytostatic or of binding to the mTOR protein. Preferably, supplemental pharmaceutical drugs include, but are not limited to, antiinflammatory drugs, corticosteriods, antithrombotics, antiplatelets, anticoagulants, antibiotics, antibacterials, antivirals, antiseptics, analgesics and anesthetics.

The term "local delivery" as used herein, refers to any drug or compound that is placed on or near a tissue surface without systemic distribution. The tissue surface includes, but is not limited to, the external skin or any internal tissue *(i.e.,* for example, the periadvential blood vessel) and/or organ surface.

The term "complementary pharmaceutical drug" as used herein, refers to any drug administered separately from a medium as contemplated by this invention. Administration of a complementary pharmaceutical drug includes, but is not limited to, oral ingestion, transdermal patch, topical, inhalation, suppository *etc*. Preferably, complementary pharmaceutical drugs include, but are not limited to, cytostatic antiproliferative drugs such as, but not limited to, sirolimus, tacrolimus, analogs of sirolimus, antiinflammatory drugs, corticosteroids, antithrombotics, antiplatelets, antibiotics, antibacterials, antivirals" analgesics and anesthetics.

The term "antiplatelets" or "antiplatelet drug" as used herein, refers to any drug that prevents aggregation of platelets or fibrin formation (*i*.*e*., for example as a prior event to adhesion formation). For example, an antiplatelet drug comprises an inhibitor of glycoprotein IIb/IIIa (GPIIb/IIIa). Further a GPIIb/IIIa inhibitor includes, but is not limited to, xemilofiban, abciximab (ReoPro^{®} cromafiban, elarofiban, orbofiban, roxifiban, sibrafiban, RPR 109891, tirofiban (Aggrastat^{®}), eptifibatide (Integrilin^{®}), UR-4033, UR-3216 or UR-2922.

The term, "antithrombins" or "antithrombin drug" as used herein, refers to any drug that inhibits or reduces thrombi formation and include, but are not limited to, bivalirudin, ximelagatran, hirudin, hirulog, argatroban, inogatran, efegatran, or thrombomodulin.

The term, "anticoagulants" or "anticoagulant drug" as used herein, refers to any drug that inhibits the blood coagulation cascade. A typical anticoagulant comprises heparin, including but not limited to, low molecular weight heparin (LMWH) or unfractionated heparin (UFH). Other anticoagulants include, but are not limited to, tinzaparin, certoparin, parnaparin, nadroparin, ardeparin, enoxaparin, reviparin or dalteparin. Specific inhibitors of the blood coagulation cascade include, but are not limited to, Factor Xa (FXa) inhibitors *(i.e.,* for example, fondaparinux), Factor IXa (FIXa) inhibitors, Factor XIIIa (FXIIIa) inhibitors, and Factor VIIa (FVIIa) inhibitors.

The term "patient", as used herein, is a human or animal and need not be hospitalized. For example, out-patients, persons in nursing homes are "patients." A patient may comprise any age of a human or non-human animal and therefore includes both adult and juveniles *(i.e.,* children). It is not intended that the term "patient" connote a need for medical treatment, therefore, a patient may voluntarily or involuntarily be part of experimentation whether clinical or in support of basic science studies.

The term "end stage renal disease" as used herein, refers to a patient having a complete or near complete failure of the kidneys to function to excrete wastes, concentrate urine, and regulate electrolytes. In particular, "end-stage renal disease" occurs when the kidneys are no longer able to function at a level that is necessary for day to day life *(i.e.,* for example, where kidney function is less than 10% of baseline). During "end-stage renal disease", the kidney function is so low that without dialysis or kidney transplantation death will occur from accumulation of fluids and waste products in the body.

The term "nephropathy" as used herein, refers to a patient having an abnormal state of the kidney, especially one associated with or secondary to some other pathological process.

The term "atherosclerotic" as used herein, refers to a patient having a condition in which fatty material is deposited along the walls of arteries (*i.e.*, for example, the renal artery). This fatty material thickens, hardens, and may eventually block the arteries. When the renal vasculature *(i.e.,* for example, the renal artery) becomes atherosclerotic, the patient may develop a condition known as, "atherosclerotic nephropathy". Atherosclerosis is just one of several types of "arterio"-sclerosis, which is characterized by thickening and hardening of artery walls, but one of skill in the art should recognize that these terms have equivalent meanings.

The term "medical device", as used herein, refers broadly to any apparatus used in relation to a medical procedure. Specifically, any apparatus that contacts a patient during a medical procedure or therapy is contemplated herein as a medical device. Similarly, any apparatus that administers a drug or compound to a patient during a medical procedure or therapy is contemplated herein as a medical device. "Direct medical implants" include, but are not limited to, urinary and intravascular catheters, dialysis catheters, wound drain tubes, skin sutures, vascular grafts and implantable meshes, intraocular devices, implantable drug delivery systems and heart valves, and the like. "Wound care devices" include, but are not limited to, general wound dressings, non-adherent dressings, burn dressings, biological graft materials, tape closures and dressings, surgical drapes, sponges and absorbable hemostats. "Surgical devices" include, but are not limited to, surgical instruments, endoscope systems (*i*.*e*., catheters, vascular catheters, surgical tools such as scalpels, retractors, and the like) and temporary drug delivery devices such as drug ports, injection needles *etc.* to administer the medium. A medical device is "coated" when a medium comprising a cytostatic or antiproliferative drug *(i.e.,* for example, sirolimus or an analog of sirolimus) becomes attached to the surface of the medical device. This attachment may be permanent or temporary. When temporary, the attachment may result in a controlled release of a cytostatic or antiproliferative drug.

The term "dialysis/apheresis catheter" as used herein, refers to any multi-lumen catheter *(i.e.,* for example, a triple lumen catheter) capable of providing a simultaneous withdrawal and return of blood to a patient undergoing a blood treatment process. Apheresis (called also pheresis) comprises a blood treatment process involving separation of blood elements that can remove soluble drugs or cellular elements from the circulation. Deisseroth et al., "Use Of Blood And Blood Products", Cancer: Principles And Practice Of Oncology, Devita, V. T. Jr. et al. Editors, Philadelphia: J. B. Lippincott Company 1989, p. 2045-2059. For example, blood is withdrawn from a donor, some blood elements (*i*.*e*., for example, plasma, leukocytes, platelets, *etc.)* are separated and retained. The unretained blood elements are then retransfused into the donor.

The term "dialysis catheter" as used herein, refers to any device capable of removing toxic substances (impurities or wastes) from the body when the kidneys are unable to do so. A dialysis catheter may comprise a single catheter having at least a dual lumen (*i.e.*, one lumen withdraws arterial blood and a second lumen returns the dialyzed blood to the venous system) or involve placing two catheters - one that is placed in an artery, and one in an adjacent vein. Dialysis catheters are most frequently used for patients who have kidney failure, but may also be used to quickly remove drugs or poisons in acute situations.

The term "peritoneal dialysis catheter" as used herein, refers to any continuous flow catheters with at least two lumens, one of which is a short lumen (used to infuse a dialysis solution into the peritoneum), and the other of which is a long coiled lumen having a plurality of openings, generally located on the inside of the coil. It is believed that peritoneal solutes enter into the coiled lumen openings and are thereby removed from the peritoneum. One hypothesis suggests that peritoneal dialysis works by using the peritoneal membrane inside the abdomen as the semipermeable membrane. Special solutions that facilitate removal of toxins may be infused in, remain in the abdomen for a time, and then drained out.

The term "fixed split-tip dialysis catheter" as used herein, refers to any catheter having at least two distinct elongated end portions that extend substantially parallel to the longitudinal axis of the catheter and are flexible to the lateral displacement of an infused fluid. It is believed that this flexibility prevents a permanent catheter tip splay that is known to injure tissue. Usually a fixed-tip dialysis catheter provides indwelling vascular access for patients undergoing long-term renal dialysis care (*i.e.*, for example, end-stage renal disease).

The term "femoral catheter" as used herein, refers to any catheter that is inserted into the femoral vein. Femoral catheters are typically provided for intermediate term blood access because the superior vena cava is relatively close to the right atrium of the heart, the minimal range of shape changes of these veins with natural movements of the patient (to minimize the damage to the vessel intima), and because of good acceptance by the patients of the skin exit on the thoracic wall. Further, the femoral veins are easy to cannulate, so that catheters of this invention may be inserted into the femoral veins at the bed side.

The term "cytostatic" refers to any drug whose antiproliferative action comprises interference with the progress of the cell cycle in the G0 or G1 phase *(i.e.,* for example, sirolimus, tacrolimus or analogs of sirolimus).

The term "endoscope" refers to any medical device that is capable of being inserted into a living body and used for tasks including, but not limited to, observing surgical procedures, performing surgical procedures, or applying medium to a surgical site. An endoscope is illustrated by instruments including, but not limited to, an arthroscope, a laparoscope, hysteroscope, cytoscope, *etc.* It is not intended to limit the use of an endoscope to hollow organs. It is specifically contemplated that endoscopes, such as an arthroscope or a laparoscope is inserted through the skin and courses to a closed surgical site.

The term, "microparticle" as used herein, refers to any microscopic carrier to which a drug or compound may be attached. Preferably, microparticles contemplated by this invention are capable of formulations having controlled release properties.

The term "PLGA" as used herein, refers to mixtures of polymers or copolymers of lactic acid and glycolic acid. As used herein, lactide polymers are chemically equivalent to lactic acid polymer and glycolide polymers are chemically equivalent to glycolic acid polymers. In one embodiment, PLGA contemplates an alternating mixture of lactide and glycolide polymers, and is referred to as a poly(lactide-co-glycolide) polymer.

The term "closure" as used herein, refers to any material that joins biological tissues or secures a surgical material to a biological tissue *(i.e.,* for example, human tissue). Such closures are known in the art to include sutures, staples, surgical wire, surgical strips *etc*. Preferably, the closure materials contemplated by the present invention are biocompatible and may or may not be bioresorbable.

The term "suture" as used herein, refers to any cord-like flexible material that joins biological tissue. Preferably, the sutures resemble sewing thread and may be looped and knotted around the tissues to ensure a proper seal.

The term "staple", as used herein, refers to any non-flexible material that joins biological tissues. Preferably, biodegradable staples are used for the fixation of soft tissues. Such staples can be used, for example, to repair vertical longitudinal full thickness tears *(i.e.* bucket-handle) of the meniscus. An example of such state of the art devices include the Absorbable Implantable Staple (United States Surgical Corporation, Norwalk, Conn.). For example, biodegradable polyhydroxyalkanoate staples can be fabricated according to the methods and procedures described by Rosenman D.C., "Spiral Surgical Tack" U.S. Patent. No. 5,728,116 (1998); Rosenman et al.*,* "Three Piece Surgical Staple" U.S. Pat. No. 5,423,857 (1995); Shlain L.M., "Methods For Use In Surgical Gastroplastic Procedure" U.S. Pat. No's. 5,345,949 & 5,327,914 (1994); Brinkerhoff et al., "Pull-Through Circular Anastomosic Intraluminal Stapler With Absorbable Fastener Means" US Pat. No. 5,222,963 (1993); Jamiolkowski, et al.*,* "Surgical Fastener Made From Glycolide-Rich Polymer Blends, U.S. Pat. No. 4,889,119 (1989); Smith et al.*,* "Surgical Fastener Made From Glycolide-Rich Polymer Blends" U.S. Pat. No. 4,741,337 (1988); Smith C.R., "Surgical Fastener Made From Polymeric Blends" US. Pat. No. 4,646,741 (1987); Schneider A.K., "Polylactide Fabric Graphs For Surgical Implantation" U.S. Pat. No. 3,797,499 (1974); and Schneider A.K., "Polylactide Sutures" U.S. Pat. No. 3,636,956 (1972)(all patents herein incorporated by reference).

The term "surgical material" as used herein refers to any device that is useful in improving the outcome of a surgical procedure. In one embodiment, a surgical material may include, but is not limited to, surgical closures, bandages, surgical mesh or surgical wraps. In another embodiment, a surgical material may include, but is not limited to, surgical instruments, surgery drapes *etc.*

The term "surgical wrap" is defined herein as a surgical material that is wrapped generally around some biological tissue at the site of a surgical procedure. The wrap could extend from a partial wrap of somewhat more or less than 180 degrees to a full wrap of somewhat more or less than a full 360-degree wrap around the tissue. To accommodate tissues having different diameters, the wrap material could be sterilized in comparatively long lengths and the surgeon could adjust it to the correct length at the time of surgery.

The term "stenosis" is defined herein as referring to any narrowing of the internal diameter of a biological tissue, such as a vessel. In particular, such narrowing is caused by phenomenon including, but not limited to, arteriosclerosis, scar tissue and/or adhesions.

The term "restenosis" is defined herein as referring to any condition wherein "stenosis", having been treated and at least partially reversed, recurs.

The term "symptom of vascular stenosis or restenosis" is defined herein as referring to any narrowing of the vasculature lumen.

The term "vascular access site" is defined herein as referring to any percutaneous insertion of a medical device into the vasculature. For example, a hemodialysis catheter placement comprises a vascular access site. Such sites may be temporary (*i*.*e*., placed for a matter of hours) or permanent (*i*.*e*., placed for days, months or years).

The term "hydrogel-based bioadhesive" as used herein, refers to any crosslinked adhesive film comprising approximately 90% water created by a self-polymerizing reaction between two precursors *(i.e.,* for example, a crosslinker molecule and a functional or multifunctional polymer). A hydrogel-based bioadhesive is created during a mixing of two aqueous (*i.e.*, liquid) media wherein a spontaneous crosslinking polymerization reaction is complete between approximately 30 minutes - 30 seconds, depending upon the types and concentrations of the two precursors. A hydrogel-based bioadhesive may degrade or hydrolyze at a predetermined rate wherein the degradation products are safely eliminated from the body.

The term "precursor" as used herein, refers to any molecule having electrophilic or nucleophilic functional groups that are water soluble, non-toxic and biologically acceptable. A precursor may comprise only nucleophilic or electrophilic functional groups, so long as both nucleophilic or electrophilic groups are used in a crosslinking reaction. For example, if a first precursor *(i.e.,* for example, a crosslinker) has nucleophilic functional groups such as amines, a second precursor (*i.e*., for example, a functional polymer) may have electrophilic functional groups such as N-hydroxysuccimides. On the other hand, if a first precursor has electrophilic functional groups such as sulfosuccinimides, then a second precursor may have nucleophilic functional groups, such as amines.

The term "functional polymer" or "multifunctional polymer" as used herein, refers to any macromolecule used as a precursor to a hydrogel-based bioadhesive that comprises two or more electrophilic or nucleophilic functional groups, such that a nucleophilic functional group on one precursor may react with an electrophilic group on another precursor to form a covalent bond. Functional polymers contemplated herein include, but are not limited to, proteins, poly(allyl amine), or amine-terminated di-or multifunctional poly(ethylene glycol). Functional polymers that are biologically inert and water soluble include, but are not limited to, polyalkylene oxides such as polyethylene glycol (PEG), polyethylene oxide (PEO), polyethylene oxide-co-propylene (PPO), co-polyethylene oxide block or random copolymers and polyvinyl alcohol (PVA); poly(vinyl pyrrolidone)(PVP), poly(amino acids); dextran and the like. The polyethers and more particularly poly(oxyalkylenes) or poly(ethylene oxide) or polyethylene oxide are especially preferred.

The term "crosslinker molecule" as used herein, refers to any small molecule having a solubility of at least 1 g/100 milliliters in an aqueous solution that comprises two or more electrophilic or nucleophilic functional groups. Preferably, a crosslinker is used as a precursor in conjunction with a functional polymer to create a crosslinked hydrogel-based bioadhesive.

The term "self-polymerizing reaction" as used herein, refers to a chemical crosslinking of two or more precursors without the provision of an external energy source. Each precursor provides either an electrophilic or nucleophilic group to the reaction such that a covalent bond is spontaneously formed. During a self-polymerizing reaction little, or no, heat production occurs.

The term "syringe" or "catheter" as used herein, refers to any device or apparatus designed for liquid administration, as defined herein. A syringe or catheter may comprise at least one storage vessel *(i.e.,* for example, a barrel) wherein a single medium resides prior to administration. A syringe or catheter comprising two or more barrels, each containing a separate medium, may mix the media from each barrel prior to administration or the media of each barrel may be administered separately. One of skill in the art will recognize that any catheter designed to perform dialysis, as defined herein, may also administer liquids.

The term "liquid" as used herein, refers to a minimally viscous medium that is applied to a surgical site by methods including, but not limited to, flowing, spraying, pouring, squeezing, spattering, squirting, and the like.

The term "dispense as a liquid" as used herein, refers to flowing, spraying, pouring, squeezing, spattering, squirting, and the like.

The term "liquid administration" as used herein, refers to any method by which a medium comprises an ability to flow or stream, either in response to gravity or by pressure-induced force.

The term "liquid spray" as used herein, refers to a liquid administration comprising the generation of finely dispersed droplets in response to pressure-induced force, wherein the finely dispersed droplets settle onto a surgical site by gravity.

The term "pourable liquids" or "flowable liquids" as used herein, refers to a liquid administration comprising the flowing or streaming of a low viscosity liquid in response to gravity. The present invention contemplates low viscosity liquids (at room temperature) ranging from between 1 and 15,000 centipoise, preferably between 1 and 500 centipoise (*i.e.*, similar to saturated glucose solution) and more preferably between 1 and 250 centipoise (*i.e.*, similar to motor oil).

The term "squeezable liquids" as used herein, refers to a liquid administration comprising the flowing or streaming of a high viscosity liquid in response to a pressure-induced force. The present invention contemplates high viscosity liquids (at room temperature) ranging from between 5,000 and 100,000 centipoise, preferably between 25,000 and 50,000 centipoise (*i.e.*, similar to mayonnaise), more preferably between 15,000 and 25,000 centipoise (*i.e.*, similar to molten glass), and more preferably between 5,000 and 15,000 centipoise (*i.e.*, similar to honey).

The term "visualization agent" as used herein, refers to any compound that improves the visibility of a medium. Visualization agents may include, but are not limited to, FD&C dyes 3 and 6, eosin, methylene blue, indocyanine green or colored dyes normally found in synthetic surgical sutures. The preferred color of a visualization agent is green or blue.

The term "vascular graft" as used herein, refers to any conduit or portion thereof intended as a prosthetic device for conveying blood and, therefore, having a blood contacting surface (*i.e.*, "luminal"). While usually in a tubular form, the graft may also be a sheet of material useful for patching portions of the circumference of living blood vessels (these materials are generally referred to as surgical wraps). Likewise, the term vascular graft includes intraluminal grafts for use within living blood vessels. The inventive grafts as such may also be used as a stent covering on the exterior, luminal or both surfaces of an implantable vascular stent.

The term "anti-adhesion drug combination" as used herein, refers to any composition comprising at least one antiproliferative drug *(i.e.,* for example, rapamycin) and at least one antiplatelet drug *(i.e.,* for example, xemilofiban). Other drugs including, but not limited to, antithrombin drugs, anticoagulant drugs or antiinflammatory drugs may also be in this combination.

The term "controlled release drug elution" as used herein, refers to any stable and quantifiable drug release from a polymer-based medium as contemplated herein.

The term "synthetic vascular graft" as used herein, refers to any artificial tube or cannula designed for insertion into a blood vessel. Such grafts may be constructed from polytetrafluoroethylene (PTFE).

The term "anti-adhesion membrane barrier" as used herein, refers to any artificial layer or device having a film-like consistency (*i*.*e*., for example, similar to plastic food wraps such as Saran Wrap^{®}). Such barriers may be applied as a pre-made wrap or may polymerize into a film following liquid administration.

The term "fibrin sheath" as used herein, refers to any encapsulation of a medical device subsequent to implantation. One hypothesis suggests that platelets and white blood cells respond to foreign substances in much the same way as an injured tissue *(i.e.,* for example, a blood vessel) and that platelet adherence, followed by fibrin encapsulation, is involved in fibrin sheath formation.

The term "non-adhesive luminal surface" as used herein, refers to any vascular graft having been constructed, or treated, that prevents platelet attachment and subsequent thrombosis formation.

### Brief Description Of The Figures

Figure 1 illustrates a surgical material to which a cytostatic anti-proliferative drug has been attached; the material is formed so that it can be wrapped around or placed on or between human tissue at the site of a surgical procedure.
Figure 2 is an enlargement of the cross section of a single strand of the mesh where the drug is embedded within the strand.
Figure 3 is an enlargement of the cross section of a single strand of the mesh where the drug is coated onto the strand.
Figure 4 is an enlargement of two strands of the mesh that have been dipped into a solution of a cytostatic anti-proliferative drug therein attaching the drug to the strands by adhesion and capillary action.
Figure 5 is a lateral cross section of cytostatic anti-proliferative surgical wrap placed around an end-to-end anastomosis of a vessel or duct.
Figure 6 is a layout view of the surgical wrap of FIG. 5.
Figure 7 is a plan view of an annular anti-proliferative surgical material for application to anastomosis.
Figure 8 is a plan view of a annular anti-proliferative surgical material for application to anastomosis, the interior of the annulus having slits to facilitate placement onto a connecting blood vessel.
Figure 9 is a cross section of cytostatic anti-proliferative surgical wrap placed at an aorta-vein graft anastomosis.
Figure 10 is a cross section of cytostatic anti-proliferative surgical wrap placed at the anastomosis of the internal mammary artery into the side of a coronary artery.
Figure 11A shows a typical plan view of a conventional suture having an attached cytostatic antiproliferative drug.
Figure 11B shows a cross-section of a conventional suture having attached cytostatic antiproliferative drug coated on the external surface as well as impregnated within the interior.
Figure 12A shows a plan view of one embodiment of an arterial end-to-end anastomosis when a surgical wrap is placed upon an artery.
Figure 12B shows a plan view of one embodiment of a healed arterial end-to-end anastomosis subsequent to when a surgical wrap is placed upon an artery.
Figure 13 shows a plan view of one embodiment of an end-to-side arteriovenous anastomosis when a surgical wrap is placed upon both the artery and the vein.
Figure 14 presents exemplary data demonstrating an equivalent monocyte adherence to PEA-comprising polymers when compared to other known polymers.
Figure 15 presents one embodiment of a polymer comprising PEA.
Figure 16 presents one embodiment of a PEA polymer comprising 4-amino TEMPO.
Figure 17 presents exemplary photomicrographs showing a lack of monocyte hyperactivation in the presence of a PEA polymer when compared to other known polymers.
Figure 18 presents exemplary data showing that one embodiment of a PEA polymer is associated with minimal interleukin 6 expression.
Figure 19 presents exemplary data showing that one embodiment of a PEA polymer is associated with minimal interleukin 1β expression.
Figure 20 presents exemplary data showing that one embodiment of a PEA polymer is associated with elevations in a naturally occuring interleukin-1 receptor antagonist expression.
Figure 21 presents exemplary photomicrographs showing minimal monocyte adherence to PEA when compared to fibrinogen.
Figure 22 presents exemplary data showing that one embodiment of a PEA polymer is associated with minimal platelet activation.
Figure 23 presents exemplary data showing that one embodiment of a PEA polymer is associated with elevated human coronary artery endothelial cell proliferation when compared to another polymer.
Figure 24 presents exemplary data showing that one embodiment of a PEA polymer has a stable and controlled enzyme-induced weight-loss rates when compared to other polymers.
Figure 25 presents exemplary data showing that one embodiment of a PEA polymer has a stable and controlled enzyme-induced reduction in molecular weight when compared to other polymers.

### Detailed Description Of The Invention

The present invention relates to devices and methods to prevent the formation of scar tissue and/or adhesions following a surgical procedure, trauma, or wound. In one embodiment, the present invention relates to medical devices comprising antiproliferative drugs (*i.e.*, for example, catheters or grafts). In another embodiment, the present invention relates to medical devices that prevent scar tissue and/or adhesion formation comprising a cytostatic antiproliferative drug in combination with other drugs including, but not limited to, antiplatelet drugs, antithrombotic drugs or anticoagulant drugs. The present invention also relates to devices and methods comprising sirolimus, tacrolimus and analogs of sirolimus to prevent the formation of scar tissue and/or adhesions following a surgical procedure. In one embodiment, the present invention relates to surgical wraps comprising sirolimus, tacrolimus and analogs of sirolimus that prevent scar tissue and/or adhesion formation following a surgical procedure.

### Combination Drug Therapy

The present invention contemplates compositions comprising antiproliferative drugs (*i.e.*, for example, the rapamycins) and/or antithrombotic drugs (*i.e.*, for example, antiplatelet, antithrombin, or anticoagulant) intended for local tissue delivery. Further, the present invention contemplates methods using these compositions to: i) prevent native and synthetic graft failure; ii) inhibit and/or reduce post-surgical adhesion formation; iii) inhibit and/or reduce fibrin sheath formation around a medical device, and iv) inhibit and/or reduce scar tissue formation. It is believed that these drug combinations have not been previously evaluated clinically. Current practice for maintaining patency of native or synthetic grafts involves utilization of thrombolytic agents (urokinase or tPA) or thrombolectomy. Ultimately, however, vascular complications require either graft replacement or graft relocation. Current practice for inhibiting post-surgical adhesion formation involves placing non-drug eluting barrier products (*i.e.*, Seprafilm^{®} or SurgiWrap^{®}) in or around the surgical site. Current practice for inhibiting fibrin sheath formation involves a mechanical stripping of the sheath from the outside of the encapsulated medical device. Current practice to prevent post-stent implantation thrombosis involves chronic systemic antiplatelet drug administration *(i.e.,* for example, aspirin and/or clopidogrel).

Excess scar tissue and/or adhesions production is a known morbidity consequence of healing from a number of types of wounds. Examples include, but are not limited to, hypertrophic burn scar tissue and/or adhesions, surgical adhesions (*i.e.*, for example, abdominal, vascular, spinal, neurological, thoracic and cardiac), capsular contracture following breast implant surgery and excess scarring and/or adhesions following eye surgery and ear surgery.

In particular, adhesion formation following surgical procedures is very common. It is known that platelets and inflammatory cells promote fibrin deposition leading to adhesion formation. Reijnen et al., "Pathophysiology Of Intra-abdominal Adhesion And Abscess Formation, And The Effect Of Hyaluronan" Br J Surg. 90:533-541 (2003). Although it is not necessary to understand the mechanism of an invention it is believed that adhesion formation is an extravascular process promoted by blood and cells escaping from a surgical site, wound, or trauma. Adhesions can form very rapidly *(i.e.,* for example, from within 7-14 days of injury) and result in severe complications for the patient, often slowing recovery or leading to additional surgical procedures. Thus, one embodiment of the present invention comprises an antiinflammatory and antithrombotic drug combination that may be very effective in reducing the incidence and severity of adhesion formation. Sirolimus (*i.e.*, rapamycin) is a known antiproliferative agent, however, this drug also possesses antiinflammatory pharmacological activity. Francischi et al., "Reduction Of Sephadex-Induced Lung Inflammation And Bronchial Hyperreactivity By Rapamycin" Braz J Med Biol Res. 10:1105-1110 (1993). Therefore, the present invention contemplates a membrane barrier material comprising an antiinflammatory *(i.e.,* for example sirolimus), an antiplatelet *(i.e*., for example, xemilofiban), an antithrombin (*i.e.*, for example, bivalirudin), or an anticoagulant *(i.e*., for example, low molecular weight heparin) drug combination that has distinct advantages over current practice using non-drug eluting barrier materials. In one embodiment, the membrane barrier material is selected from the group comprising a polymeric sheet of material or a currently marketed barrier materials including, but not limited to, Seprafilm^{®} or SurgiWrap^{®}.

Drug combination therapy involving antiproliferatives and antiplatelets is known in the medical arts. Vasculoproliferative disease (*i*.*e*., neointimal hyperplasia) has been suggested to respond after adminsitering extravascularly a rapamycin compound in combination with other antivasculoproliferative drugs. This drug combination administration is limited to impregnation into a bioresorbable matrix constructed of collagen, fibrin, or chitosan. Iyer *et al*., "Apparatus And Methods For Preventing Or Treating Failure Of Hemodialysis Vascular Access And Other Vascular Grafts" United States Patent No: 6,726,923 (2004*).* Tissue graft and organ transplant rejection may be treated with systemically administered antiplatelet drugs (glycoprotein IIb/IIIa receptor antagonists) in combination with rapamycin, tacrolimus, anticoagulants and antithrombins. Porter *et al.,* "Inhibition Of Platelet Aggregation" WO 03/090733 A1*.* Anticoagulant and antiplatelet drug combinations are known to treat conditions including acute coronary ischemic syndrome, thrombosis, thromboembolism, thrombic occlusion, restenosis, transient ischemic attack, and thrombotic stroke. Wong *et al.,* "Synergy Between Low Molecular Weight Heparin And Platelet Aggregation Inhibitors, Providing A Combination Therapy For The Prevention And Treatment Of Various Thromboembolic Disorders" WO 00/53168*;* and El-Naggar *et al.,* "Prevention And Treatment Of Thromboembolic Disorders Associated With Arterial & Venous Thrombosis" United States Patent Application Publ No: 2003/0199457A1*.* An implantable medical device *(i.e.,* limited to, stents, artificial graft, vascular sutures) is disclosed as having a coating with a least one drug that inhibits smooth muscle cell migration to prevent restenosis after implantation into a bodily organs' lumen. The antirestenosis drugs include smooth muscle cell antiproliferatives (rapamycin and everolimus), antithrombotics, and antiinflammatory drugs. Rowland *et al.,* "Drug Eluting Implantable Medical Device" United States Patent Application Publ No: 2004/0039441 A1*.* These therapies do not, however, solve the problem regarding scar tissue and/or adhesion formation either following surgery, trauma or wound. Further, these therapies do not teach one skilled in the art controlled drug release both during and after a catheter implantation such that fibrin sheath formation may be prevented. *(i.e.,* for example, during long-term dialysis).

### Antiproliferative Drugs

The present invention contemplates various embodiments wherein a medium comprising a cytostatic and antiproliferative drug *(i.e.,* sirolimus, tacrolimus and analogs of sirolimus) is applied to a surgical site or the outside of an organ with a lumen *(i.e.,* for example, extravascularly). In one embodiment, the drug reduces or prevents the formation of scar tissue and/or adhesions or tissue adhesions. The medium contemplated by this invention to deliver a specific drug, or a drug combination, to a surgical site or wound includes, but is not limited to, microparticles, gels, hydrogels, foams, bioadhesives, liquids, or xerogels. Particularly, these media are produced in various embodiments providing a controlled release of a drug such as sirolimus either singly or in a combination as according to the present invention.

Reductions in scar tissue and/or adhesion formation will be obtained if the cytostatic antiproliferative drug that is used is both cytostatic and anti-inflammatory. Improved reductions in scar tissue and/or adhesion formation will be obtained if the antiproliferative drug is combined with an antiplatelet and/or antithrombotic drug (*i*.*e*., for example, xemilofiban). Even better improved reductions in scar tissue and/or adhesion formation will be obtained if the antiproliferative-antiplatelet/antithromotic combination is further combined with an anticoagulant drug (*i.e.,* heparin or low molecular weight heparin).

In one embodiment, this invention contemplates cytostatic antiproliferative drugs such as, but not limited to, sirolimus, tacrolimus and analogs of sirolimus. For example, these drug include, but are not limited to, sirolimus, tacrolimus, everolimus, CCI-779, ABT-578, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. Other non-sirolimus related drugs may also be effective at reducing scar tissue and/or adhesion formation, including, but not limited to, antisense to c-myc and tumstatin.

Other derivatives of sirolimus comprising mono-esters and di-esters at positions 31 and 42 have been shown to be useful as antifungal agents and as water soluble prodrugs of rapamycin. Rakit S., "Acyl Derivatives OfRapamycin" U.S. Pat. No. 4,316,885 (1982); and Stella *et al.,* "Prodrugs Of Rapamycin" U.S. Pat. No. 4,650,803 (1987). A 30-demethoxy rapamycin has also been described in the literature. Vezina et al., "Rapamycin (AY-22,989), A New Antifungal Antibiotic. I. Taxonomy Of The Producing Streptomycete And Isolation Of The Active Principle" J. Antibiot. (Tokyo) 28:721-726 (1975); Sehgal et al., "Rapamycin (AY-22,989), A New Antifungal Antibiotic. II. Fermentation, Isolation And Characterization" J. Antibiot. (Tokyo) 28:727-732 (1975); Sehgal et al., "Demethoxyrapamycin (AY-24,668), A New Antifungal Antibiotic" J. Antibiot. (Tokyo) 36:351-354 (1983); and Paiva et al., "Incorporation Of Acetate, Propionate, And Methionine Into Rapamycin By Streptomycetes hygroscopicus" J. Nat Prod 54:167-177 (1991).

Numerous other chemical modifications of rapamycin have been attempted. These include the preparation of mono- and di-ester derivatives of rapamycin (WO 92/05179), 27-oximes of rapamycin (EP 467606); 42-oxo analog of rapamycin (Caufield *et al.,* "Hydrogenated Rapamycin Derivatives" U.S. Pat. No. 5,023,262 (1991)(herein incorporated by reference)); bicyclic rapamycins (Kao *et al.,* "Bicyclic Rapamycins" U.S. Pat. No. 5,120,725(1992)(herein incorporated by reference)); rapamycin dimers (Kao *et al.,* "Rapamycin Dimers" US. Pat. No. 5,120,727(1992)(herein incorporated by reference)); silyl ethers of rapamycin (Failli *et al.,* "Silyl Ethers Of Rapamycin" U.S. Pat. No. 5,120,842 (1992)(herein incorporated by reference)); and arylsulfonates and sulfamates (Failli *et al.,* "Rapamycin 42-Sulfonates And 42-(N-carboalkoxy) Sulfamates Useful As Immunosuppressive Agents" U.S. Pat. No. 5,177,203 (1993)(herein incorporated by reference)). Rapamycin was recently synthesized in its naturally occurring enantiomeric form. Nicolaou et al., "Total Synthesis Of Rapamycin" J. Am. Chem. Soc. 115: 4419-4420 (1993); Romo et al, "Total Synthesis Of (-) Rapamycin Using An Evans-Tishchenko Fragment Coupling" J. Am. Chem. Soc. 115:7906-7907 (1993); Hayward et al, "Total Synthesis Of Rapamycin Via A Novel Titanium-Mediated Aldol Macrocyclization Reaction" J. Am. Chem. Soc., 115:9345-9346 (1993).

Cytotoxic drugs such as taxol, though they are anti-proliferative, are not nearly as efficient as cytostatic drugs such as sirolimus related drugs for reducing scar tissue and/or adhesion formation resulting from a surgical procedure. Although it is not necessary to understand the mechanism of an invention, it is believed that these cytotoxic drugs, such as taxols *(i.e.,* for example, paclitaxel) act primarily by inhibiting microtubule stabilization, that is quite unlike the macrolide family *(i.e.,* for example, rapamycins), which is believed to be cytostatic by binding with the mTOR protein.

Previous attempts to solve problematic post-surgical scarring and/or adhesions using cytostatic drug therapies have used these highly cytotoxic mitosis inhibitors such as anthracycline, daunomycin, mitomycin C and doxorubin. However, no mention is made of any cytostatic antiproliferative drug such as sirolimus or similar acting drugs. Kelleher P. J., "Methods And Compositions For The Modulation Of Cell Proliferation And Wound Healing", U.S. Patent No. 6,063,396 (2000)(herein incorporated by reference). Similarly, both systemic and targeted local administration of cytotoxic antiproliferative drugs *(i.e.,* taxol) are reported to inhibit or reduce arterial restenosis. Kunz *et al.,* "Therapeutic Inhibitor Of Vascular Smooth Muscle Cells", U.S. Patent No. 5,981,568 (1999)(herein incorporated by reference). Importantly, the most preferred antiproliferative agents of Kunz *et al. (i.e.,* taxol and cytochalasin) are admitted to be cytotoxic during prolonged treatment. Kunz *et al.,* however, fails to consider the drug sirolimus or any functional sirolimus analogs for extraluminal application to reduce cellular proliferation that can result in scar tissue and/or adhesion formation.

Other attempts to reduce scar tissue and/or adhesion formation include using *beta-*emitting radioisotopes placed onto a sheet of material that irradiates the local tissue. Fischell *et al.,* "Radioisotope Impregnated Sheet Of Biocompatible Material For Preventing Scar Tissue Formation" U.S. Patent. No. 5,795,286 (1998)(herein incorporated by reference). Although radioisotopes may be effective at preventing cellular proliferation associated with adhesions, the limited shelf life and safety issues associated with radioisotopes make them less than ideal.

It is known that cellular proliferation and restenosis are reduced within angioplasty injured arteries when intraluminal vascular stents are coated with anti-proliferative drugs such as rapamycin (*i.e.*, sirolimus), actinomycin-D or taxol. Falotico *et al.,* "Drug/Drug Delivery Systems For The Prevention And Treatment Of Vascular Disease" U.S. Pat. Publ. No's. 2002/0007214A1*;* 2002/000721SA1*;* 2001/0005206 A1*;* 2001/007213A1 &.2001/0029351A1*;* and Morris *et al.,* "Method Of Treating Hyperproliferative Vascular Diseases" United States Patent No. 5,665,728 (all herein incorporated by reference). These disclosures are limited to treating hyperproliferative smooth muscle by rapamcyin administration using an intraluminal device, such as a stent.

### Antithrombotic Drugs

Platelet adherence followed by platelet aggregation is believed the first biological event to occur following any injury to a blood vessel (*i.e.,* for example, a surgical incision, trauma or wound). Although it is not necessary to understand the mechanism of an invention it is believed that platelets maintain blood hemostasis and provide a phospholipid surface for coagulation reactions to occur, thereby stabilizing a developing thrombus. Further, white blood cells *(i.e.,* leukocytes), in association with platelets, also promote coagulation reactions by expressing tissue factors that trigger the blood coagulation cascade resulting in fibrin formation and deposition. Leukocytes are also referred to in the art as inflammatory cells, thereby making the inflammatory process an integral aspect in thrombogenesis. Shebuski et al., "Role Of Inflammatory Mediators In Thrombogenesis: Perspectives in Pharmacology (PIP)" J. Pharmacol. Exp. Ther., 300:729-735 (2002).

Various embodiments of the present invention contemplate inhibiting thrombus formation. Thrombus formation inhibition may occur at various points in the blood coagulation cascade. It is generally known in the art that circulating blood platelets (3× 10⁹ cells/ml) are usually the initiating factor. Platelets may be the first to react by binding to a foreign surface or an injured tissue. Recently, GPIIb/IIIa fibrinogen receptor antagonists have been introduced as effective antiplatelet drugs. Alternatively, inhibiting fibrin formation and/or thrombus stabilization may be accomplished by administering antithrombins, heparin, low molecular weight heparin analogs or other anticoagulant drugs.

In one embodiment, the GPIIb/IIIa inhibitor is administered as a delayed release formulation, wherein the release of the inhibitor is delayed by approximately 1-3 days. Although it is not necessary to understand the mechanism of an invention; it is believed that GPIIb/IIIa acts upon a platelet receptor that activates fibrin formation.

Currently, three GPIIb/IIIa fibrinogen receptor antagonists are available commercially (Aggrastat^{®}, Integrilin^{®} and ReoPro^{®}). These drugs are administered intravenously and currently prescribed to patients: i) having angioplasty with a high risk for complications; ii) undergoing emergent percutaneous coronary intervention (*i*.*e*., for example, balloon angioplasty, atherectomy, or stent placement) starting 18-24 hours before surgery and continuing for at least an hour after surgery; and iii) with refractory unstable angina.

As mentioned above, platelets and white blood cells respond to foreign substances in much the same way as an injured tissue (*i*.*e*., for example, a blood vessel). Although it is not necessary to understand the mechanism of an invention, it is believed that platelet adherence, followed by fibrin deposition and subsequent encapsulation, is involved in fibrin sheath formation. Fibrin sheaths are known to be responsible for intravascular catheter medical complications, in particular, when using central venous and intraperitoneal dialysis catheters. Santilli, J., "Fibrin Sheaths And Central Venous Catheter Occlusions: Diagnosis And Management" Tech. in Vascular and Interventional Radiology 5:89-94 (2002).

In one embodiment, the present invention contemplates a method to prolong catheter or vascular graft (i.e., for example, a synthetic vascular graft) function comprising coating the outside surface with a drug combination comprising a GPIIb/IIIa inhibitor *(i.e.,* for example, xemilofiban) and an anticoagulant (*i.e.*, for example, a low molecular weight heparin analog) thereby preventing fibrin sheath formation. In another embodiment, the present invention contemplates a method to prolong catheter or vascular graft (i.e., for example, a synthetic vascular graft) function comprising coating the inside surface with a drug combination comprising a GPIIb/IIIa inhibitor (*i.e.*, for example, xemilofiban) and an anticoagulant (i.e., for example, a low molecular weight heparin analog) thereby preventing fibrin sheath formation.

In one embodiment, the present invention contemplates a method to prolong catheter function comprising coating the outside surface of an intravascular catheter or vascular graft (i.e., for example, a synthetic vascular graft) with a drug combination comprising antithrombins (*i.e.*, for example, bivalirudin) and an anticoagulant (*i.e.*, for example, a low molecular weight heparin analog). In another embodiment, the present invention contemplates a method to prolong catheter function comprising coating the inside surface of an intravascular catheter with a drug combination comprising antithrombins *(i.e.,* for example, bivalirudin) and an anticoagulant (i.e., for example, a low molecular weight heparin analog).

Platelets are also known to release growth factors, in particular, platelet-derived growth factor (PGDF) which promote smooth muscle cell proliferation. Schwartz et al., "Common Mechanisms Of Proliferation Of Smooth Muscle In Atherosclerosis And Hypertension" Hum Pathol. 18:240-247 (1987). For example, following stent placement in patients with coronary lesions, platelets adhere to the injured blood vessel's intraluminal surface. Subsequently, the bound platelets release growth factors that result in restenosis. Restenosis is a condition where smooth muscle cells accumulate within an injured blood vessel such that vessel blockage occurs within 3-6 months *(i.e.,* such as following an intravascular stent placement). Restenosis may be reduced with the use of drug-eluting stents, in particular with drugs such as rapamycin. Falotico *et al.,* "Drug/Drug Delivery Systems For The Prevention And Treatment Of Vascular Disease" United States Patent Application Publ. No: 2002/0016625 A1 Filed: May 7,2001. Published: February 7, 2002. However, thrombus formation following stent placement is a problem. Jeremias et al., "Stent Thrombosis After Successful Sirolimus-Eluting Stent Implantation" Circulation 109(16):1930-1932 Epub April 12 (2004). Stent technology is attempting to solve this problem using antiplatelet drug-eluting stents or grafts, but its efficacy is as yet unknown. Falotico, R., "Coated Medical Devices For The Prevention And Treatment Of Vascular Disease" United States Patent Application 2003/0216699 A1. Filed: May 7, 2003. Published; November 20, 2003.

The present invention contemplates administering a drug combination comprising an antiproliferative, an antiplatelet, an antithrombin, or an anticoagulant at, or near, an intravascular stent placement.

Platelet-mediated thrombosis is also known to complicate successful native and synthetic graft implantation. Hemodialysis vascular access sites (*infra*) or an obstructed arterial vasculature (*i*.*e*., for example, in the vascular periphery or the heart) bypass utilize these grafts. Vascular neointimal formations are known to occur in native and synthetic grafts, particularly in the venous outflow tracts. Walles et al., "Functional Neointima Characterization Of Vascular Prostheses In Human" Ann Thorac Surg. 77:864-868 (2004).

Vascular neointimal formations (*i.e.*, for example, lesions) are composed primarily of smooth muscle cells, and ultimately lead to a decreased blood flow within the grafts. Platelet-released growth factors may, in part, stimulate vascular neointimal formations. As a neointimal lesion develops, blood flow becomes more turbulent and further injury occurs, resulting in additional platelet recruitment. With additional platelet recruitment, fibrin deposition may result with complete graft failure as a probable consequence. Thus, a drug combination comprising an antiproliferative, an antiplatelet, an antithrombin, and an anticoagulant may have distinct advantages over an antiproliferative agent alone or an anticoagulant combined with just one other drug.

In one embodiment, the present invention contemplates devices and methods to administer a drug combination to a graft venous outflow tract. In one embodiment, a drug combination is administered using a controlled-release polymer-based medium or carrier. In one embodiment, the medium or carrier may be wrapped or draped around the exterior graft surface such that the drug combination diffuses to an intraluminal blood vessel surface (*i*.*e*., for example, the vaso vasorum). In one embodiment, the medium or carrier comprising at least one drug including, but not limited to, an antiproliferative drug (*i.e*., for example rapamycin), an antiplatelet drug *(i.e.,* for example, xemilofiban), an antithrombin drug *(i.e.,* for example, bivalirudin) or an anticoagulant *(i.e.,* for example, heparin). One of skill in the art will recognize that a combination of two or more drugs is intended when describing a drug combination as contemplated by the present invention.

Specific embodiments of this invention comprise treatment methods combining at least one antiproliferative drug with one or more supplemental and/or complementary pharmaceutical drugs. In one embodiment, antiproliferative drug combinations comprise supplemental and/or complementary pharmaceutical drugs including, but are not limited to, "antithrombotics" commonly known in the art as antiplatelet drugs, antithrombins, and anticoagulants. Any drug combination may be delivered locally to the surgical site before, during, or after a surgical procedure. For example, an antithrombotic and heparin combination may be used to coat intravascular catheters, or other medical devices suited to the central venous system.

In one embodiment, an antiplatelet drug includes, but is not limited to, a glycoprotein IIb/IIIa (GPIIb/IIIa) fibrinogen receptor antagonist comprising xemilofiban, cromafiban, elarofiban, orbofiban, roxifiban, sibrafiban, RPR 109891, UR-4033, UR-3216, UR-2922, abciximab, tirofiban, or eptifibatide.. Although it is not necessary to understand the mechanism of an invention, it is believed that xemilofiban is a potent antiplatelet GPIIb/IIIa fibrinogen receptor antagonist. Further, it is believed that xemilofiban hydrochloride (SC-54684A) is a prodrug (base) and undergoes rapid ester hydrolysis into a pharmacologically active acid metabolite (*i*.*e*., for example, SC-54701A). Further, one having skill in the art should realize that antiplatelet GPIIb/IIIa fibrinogen receptor antagonists are also known as platelet GPIIb/IIIa receptor antagonists.

In one embodiment, an antithrombin includes, but is not limited to, bivalirudin, ximelagatran, hirudin, hirulog, argatroban, inogatran, efegatran, or thrombomodulin.

In one embodiment, an anticoagulant comprises heparin. In one embodiment, an anticoagulant comprises a low molecular weight heparin (LMWH). In another embodiment, an anticoagulant comprises an unfractionated heparin (UFH). In another embodiment, an anticoagulant includes, but is not limited to, tinzaparin, certoparin, parnaparin, nadroparin, ardeparin, enoxaparin, reviparin or dalteparin. In one embodiment, an anticoagulant includes, but is not limited to, Factor Xa (FXa) inhibitors *(i.e.,* for example, fondaparinux), Factor IXa (FIXa) inhibitors, Factor XIIIa (FXIIIa) inhibitors, and Factor VIIa (FVIIa) inhibitors.

### Polymer-Based Media

Another embodiment of the present invention contemplates coating a medical device with a medium or carrier comprising sirolimus, tacrolimus or an analog of sirolimus. A medical device is "coated" when a medium comprising a cytostatic or antiproliferative drug *(i.e.,* for example, sirolimus or an analog of sirolimus) becomes attached to the surface of the medical device. For example, such attachment includes, but is not limited to, surface adsorption, impregnation into the material of manufacture, covalent or ionic bonding and simple friction adherence to the surface of the medical device.

Carriers or mediums contemplated by this invention may comprise a polymer including, but not limited to, gelatin, collagen, cellulose esters, dextran sulfate, pentosan polysulfate, chitin, saccharides, albumin, fibrin sealants, synthetic polyvinyl pyrrolidone, polyethylene oxide, polypropylene oxide, block polymers of polyethylene oxide and polypropylene oxide, polyethylene glycol, acrylates, acrylamides, methacrylates including, but not limited to, 2-hydroxyethyl methacrylate, poly(ortho esters), cyanoacrylates, gelatin-resorcin-aldehyde type bioadhesives, polyacrylic acid and copolymers and block copolymers thereof; poly (L- lactide) (PLA), 75/25 poly(DL-lactide-co-E-caprolactone), 25/75 poly (DL-lactide-co-E- caprolactone), poly(ε-caprolactone) (PCL), collagen, polyactive, and polyglycolic acid (PGA); polytetrafluoroethylene, polyurethane, polyester, polypropylene, polyethylene, polydioxanone (PDO), and silicone. Other polymers may include, but are not limited to, cellulose acetate, cellulose nitrate, silicone, cross-linked polyvinyl alcohol (PVA) hydrogel, cross-linked PVA hydrogel foam, polyurethane, polyamide, styrene isobutylene-styrene block copolymer (Kraton), polyethylene teraphthalate, polyurethane, polyamide, polyester, polyorthoester, polyanhidride, polyether sulfone, polycarbonate, polypropylene, high molecular weight polyethylene, polytetrafluoroethylene, or other biocompatible polymeric material, or mixture of copolymers thereof; polyesters such as, polylactic acid, polyglycolic acid or copolymers thereof, a polyanhydride, polycaprolactone, polyhydroxybutyrate valerate or other biodegradable polymer, or mixtures or copolymers, extracellular matrix components, proteins, collagen, fibrin or other bioactive agent, or mixtures thereof.

The medium can be selected from a variety of polymers. However, the media should be biocompatible, biodegradable, bioerodible, non-toxic, bioabsorbable, and with a slow rate of degradation. Biocompatible media that can be used in the invention include, but are not limited to, poly(lactide-co-glycolide), polyesters such as polylactic acid, polyglycolic acid or copolymers thereof, polyanhydride, polycaprolactone, polyhydroxybutyrate valerate, and other biodegradable polymer, or mixtures or copolymers, and the like. In another embodiment, the naturally occurring polymeric materials can be selected from proteins such as collagen, fibrin, elastin, and extracellular matrix components, or other biologic agents or mixtures thereof.

Polymer media used with the coating of the invention such as poly(lactide-co-glycolide); poly-DL-lactide, poly-L-lactide, and/or mixtures thereof are of various inherent viscosities and molecular weights. For example, in one embodiment of the invention, poly(DL lactide-co-glycolide) (DLPLG, Birmingham Polymers Inc.) is used. Poly(DL-lactide-co-glycolide) is a bioabsorbable, biocompatible, biodegradable, non-toxic, bioerodible material, which is a vinylic monomer and serves as a polymeric colloidal drug carrier. The poly-DL-lactide material is in the form of homogeneous composition and when solubilized and dried, it forms a lattice of channels in which pharmaceutical substances can be trapped for delivery to the tissues.

The drug release kinetics of any coating on any device contemplated by some embodiments of the present invention can be controlled depending on the inherent viscosity of the polymer or copolymer used as the matrix and the amount of drug in the composition. The polymer or copolymer characteristics can vary depending on the inherent viscosity of the polymer or copolymer. For example, in one embodiment of the invention using poly(DL-lactide-co-glycolide), the inherent viscosity can range from about 0.55 to 0.75 (dL/g). Poly(DL-Lactide-co-Glycolide) can be added to the coating composition from about 50 to about 99% (w/w) of the polymeric composition. A poly(DL-lactide-co-glye- olide) polymer coating amy deform without cracking, for example, when the coated medical device is subjected to stretch and/or elongation and undergoes plastic and/or elastic deformation. Therefore, polymers which can withstand plastic and elastic deformation such as poly(DL-lactide-co-glycolide) acid-based coats, have advantageous characteristics over known polymers. The rate of dissolution of the media can also be controlled by using polymers of various molecular weight. For example, for slower rate of release of the pharmaceutical substances, the polymer should be of higher molecular weight. By varying the molecular weight of the polymer or combinations thereof, a preferred rate of dissolution can be achieved for a specific drug. Alternatively, the rate of release of pharmaceutical substances can be controlled by applying a polymer layer to the medical device, followed by one or more than one layer of drugs, followed by one or more layers of the polymer. Additionally, polymer layers can be applied between drug layers to decrease the rate of release of the pharmaceutical substance from the coating.

Further, the malleability of the coating composition of some embodiments of the present invention can be further improved by varying the ratio of lactide to glycolide in the copolymer. That is, the ratio of components of the polymer can be adjusted to make the coating more malleable and to enhance the mechanical adherence of the coating to the surface of the medical device and aid in the release kinetics of the coating composition. In this embodiment of the invention, the polymer can vary in molecular weight depending on the rate of drug release desired. The ratio of lactide to glycolide can range, respectively, from about 50-85% to 50-15% in the composition. By adjusting the amount of lactide in the polymer, the rate of release of the drugs from the coating can also be controlled.

GPIIb/IIIa inhibitors may be attached to a medical device in a number of ways and utilizing any number of biocompatible materials (*i.e*., polymers). Different polymers are utilized for different medical devices. For example, a ethylene-co-vinylacetate and polybutylmethacrylate polymer is utilized with stainless steel. Falotico et al., United States Patent Application, 2002/0016625. Other polymers may be utilized more effectively with medical devices formed from other materials, including materials that exhibit superelastic properties such as alloys of nickel and titanium. In one embodiment, drugs such as, but not limited to, GPIIa/IIIb inhibitors, sirolimus, tacrolimus or analogs of sirolimus are directly incorporated into a polymeric medium and sprayed onto the outer surface of a catheter such that the polymeric spray becomes attached to said catheter. In another embodiment, said drug will then elute from the polymeric medium over time and enter the surrounding tissue. In one embodiment, said drug is expected to remain attached on the catheter for at least one day up to approximately six months. One of skill in the art will recognize that any drug may preferentially integrate with a polymer-based medium as either a base or acid formulation. In one embodiment, an antiplatelet drug *(i.e.,* for example, xemilofiban) is converted to an acid formulation prior to integration into a polymer-based medium.

In one embodiment, the present invention contemplates a method of preventing post-operative surgical adhesions of tissue, protecting tissue and/or preventing tissue damage during surgery. In one embodiment, the method provides the tissue surfaces involved in the surgery with a wet coating of a physiologically acceptable aqueous solution of a hydrophilic polymeric material *(i.e.,* for example, hyaluronic acid) prior to manipulation of the tissue during the surgery. Goldberg *et al.,* "Method And Composition For Preventing Surgical Adhesions And Tissue Damage" United States Patent No: 6,010,692 (2000)(herein incorporated by reference). Hyaluronic acid comprises a linear chain of about 2500 repeating disaccharide units in specific linkage, each composed of an N-acetylglucosamine residue linked to one glucuronic acid residue. In one embodiment, the hyaluronic acid polymeric material further comprises drugs including, but not limited to, antiproliferative drugs (*i*.*e*., for example, rapamycin), antiplatelet drugs *(i.e.,* for example, xemilofiban), antithrombin drugs, anticoagulant drugs (*i.e.*, for example, heparin) or antiinflammatory drugs. In one embodiment, the hydrophilic polymeric material comprises a commercially available product (*i.e.*, for example, Seprafilm^{®}).

### Membrane Barriers

Reducing postoperative adhesions is known when using a drapable, conformable adhesion barrier fabric constructed of a bioresorbable material, such as oxidized regenerated cellulose (ORC) knitted fabric. Linsky *et al.,* "Heparin-Containing Adhesion Prevention Barrier And Process" United States Patent No: 4,840,626 (1989)(herein incorporated by reference). In one embodiment, a membranous adhesion barrier comprises a fabric of oxidized regenerated cellulose impregnated with heparin and characterized by having a porosity as defined by open area of 12 to 20 percent and a density of from about 8 to 15 mg/cm². Linsky *et al*., "Method And Material For Prevention Of Surgical Adhesions" United States Patent No. 5,002,551 (1991)(herein incorporated by reference). In one embodiment, the membrane barrier is prepared from 60 denier, 18 filament bright rayon yarn knitted on a 32 gauge 2 bar warp knitting machine. In another embodiment, the membrane barrier is a commercially available product *(i.e.,* for example, Interceed^{®}, Johnson & Johnson). In another embodiment, the heparin-ORC membrane barrier further comprises a drug combination comprising antiproliferative drugs, antiplatelet drugs or antithrombin drugs. Other commercially available ORC products may also be coated with embodiments of the present invention *(i.e.,* for example, Surgicel^{®}). Although it is not necessary to understand the mechanism of an invention, it is believed that heparin acts as an adhesion-preventing medicament upon incorporation into the polymer coatings of the present invention.

In one embodiment, the present invention contemplates an improved anti-adhesion polymer membrane barrier wherein the polymer membrane barrier comprises a drug-eluting medium *(i.e.,* for example, a controlled release medium). Polymer membrane barriers are currently commercially available that are compatible with the improvements described herein. *(i.e.,* for example, SurgiWrap^{®}). One of skill in the art will recognize that similar anti-adhesion polymer membrane barriers compatible with the improvements described herein may be constructed from other compositions comprising polymers including, but not limited to, gelatin-riboflavin polymers crosslinked *in situ* with ultraviolet light, poly(ethylene oxide-copropylene oxide) polymers, chitosan-poly(ethylene glycol) polymers, or pourable (i.e., for example, flowable) sodium alginate polymers.

In one embodiment, the present invention contemplates a method for administering a hydrogel-based bioadhesive to a surgical site, comprising: a) providing; i) a surgical site *(i.e.,* for example, open or closed); ii) a twin-barrel syringe or catheter comprising; I) a first barrel containing a first aqueous medium comprising sirolimus and analogs of sirolimus and a functional polymer; and II) a second barrel containing a second aqueous medium comprising a small crosslinker molecule; b) contacting the first and second mediums onto a surgical site *(i.e.,* for example, open or closed) under conditions such that the first and second aqueous mediums become mixed; and c) crosslinking the first and second mediums initiated by a self-polymerizing reaction to form a bioadhesive layer on the surgical site. In one embodiment, the first and second mediums are sprayed on the surgical site. In one embodiment, the first and second mediums are sequentially contacted with the surgical site. In another embodiment, the first and second mediums are mixed prior to contacting the surgical site. Preferably, the mixing occurs on a surface of a surgical site to form a crosslinked adhesive barrier; exemplary crosslinker molecules and functional polymers include, but are not limited to, components comprising DuraSeal^{™} or SprayGel^{™} (Confluent Surgical, Waltham. MA). Preul et al., "Toward Optimal Tissue Sealants For Neurosurgery: Use Of A Novel Hydrogel Sealant In A Canine Durotomy Repair Model", Neurosurgery 53:1189-1199 (2003). In one embodiment, sirolimus and analogs of sirolimus are phase-separated in the first aqueous medium. In one embodiment, the first aqueous medium further comprises a supplemental or complementary drug selected from an antiplatelet drug, an antithrombin drug, an anticoagulant drug, or an antiinflammatory drug. In one embodiment, the phase separation comprises an oil-water mixture. In another embodiment, the phase separation comprises microparticles as described herein. In one embodiment, the crosslinked adhesive barrier forms a controlled release medium.

Another embodiment of the present invention contemplates a hydrogel-based bioadhesive comprising: i) a first medium comprising sirolimus and analogs of sirolimus and a functional polymer and ii) a second medium comprising a small crosslinker molecule. In one embodiment, the first medium further comprises a supplemental or complementary drug selected from an antiplatelet drug, an antithrombin drug, an anticoagulant drug, or an antiinflammatory drug. In one embodiment, the crosslinker molecule includes, but is not limited to, ethoxylated glycerols, inositols, trimethylolpropanes, succinates, glutarates, combinations of 2 or more esters (i.e., for example, glycolate/2-hydroxybutyrate or glycolate/4-hydroxyproline). In one embodiment, the functional polymer includes, but is not limited to, polyethylene oxide or polyethylene glycol. Preferably, this hydrogel-based bioadhesive forms a biocompatible crosslinked polymer from water soluble precursors having electrophilic and nucleophilic groups capable of reacting and crosslinking in situ. Pathak *et al*., "Biocompatible Crosslinked Polymers" United States Patent No. 6,566,406 (herein incorporated by reference). In one embodiment, the crosslinked polymers are biodegradable or bioresorbable. Certain embodiments are contemplated that provide biodegradable crosslinkages that allow degradation or resorption within a predetermined period of time (*i.e.*, for example, by chemically or enzymatically hydrolyzable crosslinkages). Examples of such chemically hydrolyzable linkages include, but are not limited to, polymers, copolymers and oligomers of glycolide, (dl)-lactide, (1)-lactide, caprolactone, dioxonone or trimethylene carbonate. Examples of such enzymatically hydrolyzable linkages include, but are not limited to, peptide linkages cleavable by metalloproteinases or collagenase. Over time, the hydrogel-based bioadhesive liquefies to form water-soluble materials that are absorbed and readily cleared from the body (*i*.*e*., for example, by renal action). The crosslinking reactions preferably occur in aqueous solution under physiological conditions. In one embodiment, the crosslinking reactions occur "in situ", meaning they occur at local sites such as organs or tissues in a living animal or human body. In one embodiment, the crosslinking reactions do not release a substantial heat of polymerization. In one embodiment, the crosslinking reaction is completed within 10 minutes, preferably within 2 minutes, more preferably within one minute and most preferably within 30 seconds.

### Medical Device Coatings

Anti-adhesion drug combination coatings contemplated by various embodiments of the present invention may comprise polymers having a covalent attachment to inner and outer surfaces of a medical device. In one embodiment, the coating provides versatile surface characteristics, such as lubricity, therapeutic loading and duration of therapeutic efflux. In another embodiment, the coating comprises characteristics including, but not limited to, lubricious, hydrophilic, flexible loading capabilities, controllable therapeutic release kinetics, an inner and outer lumen coating that does not significantly alter the diameter of the device, and is biocompatible. In one embodiment, a drug combination coating further comprises a silver-based antimicrobial composition effective against pathogenic bacteria *(i.e.,* for example, *Staphylococcus aureus* and *Pseudomonas).*

In one embodiment, an anti-adhesion drug combination coating composition comprises a commercially available high-quality hydrophilic polymer that is covalently bonded to a polymeric invasive medical device *(i.e.,* for example, Covalon Technologies Inc., Toronto Canada). Although it is not necessary to understand the mechanism of an invention it is believed that the coating results in improved biocompatibility and functionality by reducing the coefficient of static friction of a medical device polymer surface including, but not limited to, silicone, polyurethane, or polyvinyl chloride. Further, it is believed that the surface coating acts as a repository for a controlled efflux a drug combination composition at the site of device insertion or application. In one embodiment, an anti-adhesion drug combination is applied after coating the medical device. In one embodiment, coated medical devices include, but are not limited to, catheters, peritoneal dialysis catheters, hemodialysis catheters, wound drains, central venous lines, other tubular medical devices, and various wound dressings and skin coverings.

The present invention contemplates a method comprising dip-coating a medical device with a polymer-based drug combination medium and polymerizing the polymer-based drug combination by exposure to ultraviolet light. In one embodiment, the polymerization is a low-energy, surface modification process applicable to polymers including, but not limited to, silicone, polyurethane, or polyvinyl chlorides. Although it is not necessary to understand the mechanism of an invention, it is believed that when a polymer is activated by ultraviolet light, initiator reagents yield highly reactive intermediate molecules that remove a hydrogen atom from the polymer surface. Further, it is believed that the reactive polymer surface now allows monomers in solution to form carbon-carbon or carbon-nitrogen bonds with the polymer device surface by a chain reaction mechanism that also causes the monomers in solution to form a covalent polymer coating. In one embodiment, the initiator intermediates are highly reactive and facilitate creating covalently bound coatings. In another embodiment, the drug combination is integrated after polymer-based medium formation. In another embodiment, the drug combination polymer coating further comprises hydrated and dehydrated collagen. For example, these collagen-based polymer medium devices include, but are not limited to, topical and implantable surgical sheets of material *(i.e.,* surgical wraps, sutures, gauzes *etc.)* or three-dimensional scaffolds useful for skin or tissue regeneration following trauma or burns.

One of skill in the art will recognize that polymers for coating medical devices (*i*.*e*., for example, vascular grafts and intravascular catheters) include, but are not limited to, polyvinyl pyrrolidone, poly(acrylic acid), poly(vinyl acetamide), poly(propylene glycol), poly(ethylene co-vinyl acetate), poly(n-butyl methacrylate) or poly(styrene-b-isobutylene-b-styrene).

One embodiment of this invention contemplates a composition that slowly releases drugs *(i.e.,* for example, a cytostatic antiproliferative drug) in a controlled manner to reduce the formation of scar tissue and/or adhesions following a surgical procedure, trauma, or wound. In one embodiment, cytostatic drugs may be attached to medical devices comprising a surgical material and a medium, wherein said devices include, but are not limited to, catheters, grafts, meshes, wraps or closures. In another embodiment, the cytostatic drug may be combined with other drugs including, but not limited to, antiplatelet drugs, antithrombotic drugs, anticoagulant drugs or antiinflammatory drugs. In one embodiment, the antiplatelet drug comprises xemilofiban, cromafiban, elarofiban, orbofiban, roxifiban, sibrafiban, RPR 109891, UR-4033, UR-3216, UR-2922, abciximab, tirofiban, or eptifibatide. In another embodiment, the antiplatelet drug comprises SC-54701A, an acid xemilofiban metabolite. The medium may comprise polymers and/or copolymers that slowly elute drugs (for a time of at least one day) from the medical device onto which the medium is attached. In one embodiment, the medium provides a controlled release of cytostatic anti-proliferative drugs, such as sirolimus, tacrolimus and analogs of sirolimus. In another embodiment, other drugs including, but not limited to, antiplatelet drugs, antithrombotic drugs or anticoagulant drugs may also be released from the medium or device in a controlled manner. Alternatively, the drug may be attached directly to a device and subsequently released. Although it is not necessary to understand the mechanism of a successful invention, it is believed that sirolimus-like drugs interfere with the initiation of mitosis by means of interaction with the mTOR protein complex formation and cyclin signaling. Furthermore, it-is believed that these drugs prevent the initiation of DNA replication by acting on cells in close proximity to the mesh from which the drug slowly elutes as very early cell cycle mitosis inhibitors that act at or before the S-phase of cellular mitosis.

The present invention contemplates a medium that has the capability of providing controlled release of drugs. For example, liposomes, microparticles, gels, hydrogels, xerogels, foams are known media having compositions compatible with controlled release characteristics. Specifically, liposomes and microparticles may provide controlled release of a drug by varying, for example, polymer composition, concentration, physical size or physical shape. Gels and hydrogels may comprise controlled release liposomes or microparticles. Alternatively, the polymer composition or concentration of a gel or hydrogel may result in the production of a micellular gel structure wherein the dissolution of the gel itself is responsible for the controlled release of the attached drug. Furthermore, foams may comprise liposomes or microparticles that allow the medium to provide controlled release characteristics.

In one embodiment, the present invention contemplates a sirolimus hydrogel polymer coating on a stainless steel medical device (*i*.*e*., for example, a permanent implant). Preferably, a stainless steel implant is brush coated with a styrene acrylic aqueous dispersion polymer (55% solids) and dried for 30 minutes at 85°C. Next, this polymer surface is overcoated with a controlled release hydrogel composition consisting of:

| | |
|---|---|
| Polyvinyl pyrrolidone (PVP) | 9.4 gm |
| Ethanol | 136.1 gm |
| Butyrolactone | 30.6 gm |
| 0.0625% nitrocellulose in cyclohexanone | 3.8 gm |
| Sirolimus (dissolved in olive oil) | 10 mg/ml |

The coating is then dried for 25 hours at 85°C prior to use. It is not intended that the present invention be limited by the above sirolimus concentration. One skilled in the art should realize that that various concentrations of sirolimus may be used such as, but not limited to, 0.001 - 10 mg/ml, preferably 0.1 - 5 mg/ml, and more preferably 0.001 - 1 mg/ml.

In another embodiment, a multiple layering of non-erodible polymers may be utilized in conjunction with sirolimus. Preferably, the polymeric medium comprises two layers; a inner base layer comprising a first polymer and the incorporated sirolimus and an outer second polymer layer acting as a diffusion barrier to prevent the sirolimus from eluting too quickly and entering the surrounding tissues. In one embodiment, the thickness of the outer layer or top coat determines the rate at which the sirolimus elutes from the medium. Preferably, the total thickness of the polymeric medium is in the range from about 1 micron to about 20 microns or greater. Another embodiment of the present invention contemplates spraying or dipping a polymer/sirolimus mixture onto a catheter.

### Di-Amino Acid Polymers

In one embodiment, the present invention contemplates a composition comprising a di-amino acid polymer (i.e., for example, a poly(ester amide); PEA), an antiproliferative drug (i.e., for example, sirolimus, tacrolimus and analogs of sirolimus), and another drug including, but not limited to, antiplatelet drugs, antithrombotic drugs, or anticoagulant drugs. In one embodiment, the di-amino acid polymer comprises a family of biodegradable polymers composed of naturally occuring amino acids and other nontoxic building blocks.

Di-amino acid polymers may be prepared under mild solution polymerization conditions, are devoid of toxic catalysts, have reproducible molecular weights, and exhibit excellent blood and tissue compatibility. For example, PEA may be made by synthesizing monomers of two alpha-amino acids, (i.e., for example, L-leucine and L-lysine) with a diol (i.e., for example, hexanediol) and a diacid (e.g., i.e., for example, sebacic acid (1,8-octanedicarboxylic acid).

*In vivo* PEA biocompatibility was tested by implanting either PEA polymer-coated stents or bare metal stents into porcine coronary arteries. No differences in stent induced restenosis were seen. Specifically stenotic diameter, injury score, and stenotic area were not different between the two groups. This study suggests that the PEA polymers are suitable for implantation. Lee et al., "In-vivo biocompatibility evaluation of stents coated with a new biodegradable elastomeric and functional polymer" Coron Artery Dis. 13:237-41 (2002).

In one embodiment, the present invention contemplates a di-amino acid polymer comprising at least one carboxyl group. In one embodiment, a lysine amino acid comprises the carboxyl group. In one embodiment, the carboxyl group attaches a drug. In one embodiment, the drug may be selected from the group comprising sirolimus, tacrolimus, analogs of sirolimus, antiplatelet drugs, antithrombotic drugs, or anticoagulant drugs.

In one embodiment, the present invention contemplates a di-amino acid polymer comprising an antioxidant. In one embodiment, the antioxidant comprises tempamine (4-amino-2,2,6,6-tetramethylpiperidine-N-oxyl; also known as 4-amino TEMPO). In one embodiment, a PEA is conjugated to PEA-4-amino-TEMPO (i.e., for example, PEA-TEMPO).

Pharmaceutical grade biodegradable polymers (i.e., for example, polylactic acid and polyglycolic acid) are known in the medical arts, but have proven inadequate to provide sustained site-specific drug delivery applications due to their degradation characteristics. Specifically, these polymers degrade by hydrolysis, making them inherently unstable in biologic conditions. This degradation by water results in bulk erosion, resulting in grossly ineffective drug delivery capabilities. Consequently, medical devices containing these polymers provide an erratic release of pharmaceutical agents within the body, both in terms of the quantity of agent released, as well as the release horizon.

In one embodiment, the present invention contemplates a PEA polymer having no hydrolytic degradation, wherein an incorporated drug elutes from the polymer. In another embodiment, a PEA polymer contacting an enzymatic solution (i.e., for example, chymotrypsin or an esterase enzyme) degrades uniformly and linearly. Although it is not necessary to understand the mechanism of an invention, it is believed that a uniform and linear degradation profile will provide an effective and controlled drug delivery.

In one embodiment, the present invention contemplates a PEA copolymer capable of promoting a natural healing response. PEA polymer are biocompatible following exposure of human peripheral blood monocytes to PEA, PEA-TEMPO, 50:50 poly(D,L-lactide-co-glycolide) (PLGA), poly(n-butyl methacrylate) (PBMA) and tissue culture-treated polystyrene (TCPS). Also, human coronary artery endothelial cells (EC) were grown or human platelets were exposed to PEA, PEA-TEMPO and non-degradable polyethylene-co-vinyl acetate (PEVAc)/PBMA and showed no toxic effects.

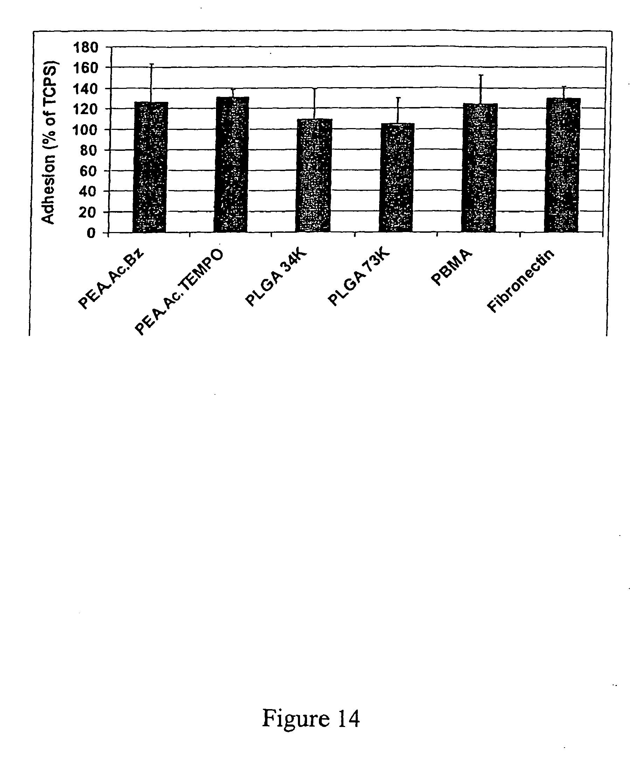

PEA polymer surfaces modulate the morphology and quantity of adherent monocytes. For example, monocytes attached to PEA and/or PEA-TEMPO polymers differentiated into macrophages which fused to form multinucleated cells at an equivalent rate to a control, non-activating TCPS, and other polymers. For example, human monocytes may be seeded into wells. After a twenty-four hour incubation adhesion may be monitored by quantifying intracellular adenosine triphosphate levels. Figure 14. Adherent monocytes were also assayed for pro-inflammatory activation.

Interleukin-6 (IL-6) is known to be secreted by activated monocytes and may induce secretion of additional pro-inflammatory cytokines. Monocytes attached to PEA and/or PEA-TEMPO secreted reduced levels of IL-6 when compared to PLGA and PBMA.

Human coronary artery endothelial cells may be attached to PEA and/or PEA-TEMPO to determine natural healing property promotion capability. Endotheial cell proliferation on PEA is known to be 4-fold higher than on PEVAc/PBMA.

Hemocompatibility can be determined by contacting PEA polymer with freshly isolated human platelets for 30 minutes. ATP release, a measure of activation, from platelets on PEA are known to be 2-fold lower than platelets on PEVAc/PBMA.

*In vitro* assessments of the tissue compatibility of biodegradable amino acid-based polymers (i.e., for example, PEA polymers) suggest that these polymers may promote the natural healing response by attenuating the pro-inflammatory reaction and promoting re-endothelialization. In addition, platelet activation suppression suggests that polymers comprising PEA are hemocompatible.

Poly(ester amide) (PEA) polymers have numerous advantages over other well-known biodegradable polymers including, but not limited to:
i) Programmability-PEA polymer components can be changed to customize biological and physical properties;
ii) Functionalization - pharmaceutical compounds (i.e., for example, sirolimus, tacrolimus, analogs of sirolimus, antiplatelets, antithrombotics, anticoagulants) can be covalently conjugated to the polymer backbone via amino acid functional groups. Alternatively, such pharmaceutical compounds may be incorporated (i.e., for example, by non-covalent interactions) within the polymer matrix, wherein the compounds elute in a controlled mannner;
iii) Elasticity - a PEA polymer may elongate greater than 300%;
iv) Uniform surface degradation - provides a controlled release of attached drugs
v) Enzymatic biodegradation - enzymatic attack of the amino acid-like ester and amide bonds
vi) Proven blood, cell and tissue biocompatibility - in vitro, pre-clinical and clinical studies.

Although it is not necessary to understand the mechanism of an invention, it is believed that PEA and/or PEA-TEMPO polymers provide biodegradable polymers suitable for clinical cardiovascular therapies. It is also believed that: i) PEA polymers promote the natural healing response by attenuating the pro-inflammatory reaction and promoting re-endothelialization (i.e., for example, monocytes adhere to PEA surfaces but do not generate a pro-inflammatory response); ii) endothelial cells preferentially adhere and proliferate on PEA polymers as compared to smooth muscle cells; iii) PEA polymers suppress platelet adhesion, aggregation, and activation; and iv) an enzyme-driven, PEA surface erosion biodegradation may be controlled by enzymatic means.
DeFife et al., "Poly(ester amide) promotes hemocompatibility and tissue compatibility" TCT 2004, Washington, D.C*.*

In one embodiment, the present invention contemplates a PEA polymer that can be programmed for different drug delivery applications. In one embodiment, polymer programming comprises obtaining desired physical properties by selecting different components that make up the polymer backbone. Alternatively, different methods of making the PEA polymer are contemplated. For example, PEA materials can be combined with drugs by mechanical mixing that allows the release of the drug to be controlled by diffusion (i.e., for example, by non-covalent interactions). Alternatively, drugs can be conjugated to the polymers by covalent attachment and released by polymer biodegradation once they reach their targets.

### Drug Delivery Devices

Many drug delivery means are known in the art including, but not limited to, sheets of material, catheters, syringes, foams, gels, sprays *etc.* Fischell *et al.,* United States Patent Publication No: 2004/0018228 A1 (herein incorporated by reference). The methods of the present invention are exemplified by the following description of various medical device embodiments. These illustrations are not intended to limit the scope of the invention but are only intended as examples.

### Dialysis Catheters

One embodiment of the present invention comprises a method to reduce and/or prevent fibrin sheath formation on dialysis catheters. Another embodiment comprises a method to coat a catheter with a drug and/or drug combinations as contemplated herein.

In one embodiment, the present invention contemplates an improved dialysis/apheresis catheter comprising an anti-adhesion drug combination (*i*.*e*., for example, a GPIIb/IIIa inhibitor and an antiproliferative drug). Dialysis/apheresis and peritoneal dialysis catheters are used in both acute and chronic clinical applications. In one embodiment, a dialysis/apheresis catheter coating comprises a drug combination including an antiproliferative, antiplatelet, antithrombin or an anticoagulant that inhibits fibrin sheath formation. It is known that most dialysis/apheresis catheters comprise multilumens (3 or 4 lumen) that may be used simultaneously (*i.e.*, thereby allowing a withdrawal and return of equal amounts of blood). In one embodiment, these lumens match flow resistance between a designated inflow lumen and a designated outflow lumen, and supports a high exchange flow rate for long-term placements. Loggie B.W., "Multi-Lumen Catheter System Used In A Blood Treatment Process" United States Patent 6,126,631 (2000)(herein incorporated by reference).

In another embodiment, the present invention contemplates an improved dialysis catheter comprising an anti-adhesion drug combination coating. Martin *et al.,* "Triple Lumen Catheter" United States Patent No: 5,195,962 (1993)(herein incorporated by reference). Commercially available dialysis catheters include, but are not limited to, Vas-Cath^{®} or Hickman^{®} catheters (Bard Access Systems). One skilled in the art will recognize that these catheters are useful for acute and chronic conditions, provide optimal flow rates with a small insertion profile, are available in a variety of French sizes, single- or dual-lumen configurations, and have straight or precurved configurations. In one embodiment, a dialysis catheter coating comprises a drug combination including an antiproliferative, antiplatelet, antithrombin or an anticoagulant that inhibits fibrin sheath formation. In another embodiment, the dialysis catheter comprises a tissue in-growth cuff (*i.e.*, for example, SureCuff^{®}), that optionally, may comprise an antimicrobial cuff *(i.e.,* for example, VitaCuff^{®}), both of which are coated with an anti-adhesion drug combination.

In another embodiment, the present invention contemplates an improved peritoneal dialysis catheter (*i.e.*, for example, Tenckhoff^{™}, Bard Access Systems) comprising an anti-adhesion drug combination. In one embodiment, the peritoneal dialysis catheter comprises either one or two tissue in-growth cuffs *(i.e.,* for example, SureCuff^{®}) and/or an antimicrobial cuff *(i.e.,* for example, VitaCuff^{®}). Although it is not necessary to understand the mechanism of an invention, it is believed that peritoneal dialysis is a continuous flow technique which utilizes a certain amount of fluid *(i.e.,* for example, a dialysate) which is constantly infused into the abdomen. Continuous flow peritoneal dialysis previously known in the art has utilized two single lumen peritoneal dialysis catheters or a modified large bore hemodialysis catheter. The inflow and uptake catheters enable the dialysate inflow and outflow to remain constant. However, high dialysate flow rates and re-circulation due to channeling or poor mixing inside the peritoneal cavity are problems associated with continuous flow peritoneal dialysis and may result in tissue injury or trauma. In one embodiment, the present invention contemplates an anti-adhesion drug composition attached to a continuous flow peritoneal dialysis catheter that effectively allows the dialysate to mix into the peritoneum while reducing trauma to the peritoneal walls. In the continuous flow peritoneal dialysis technique, the peritoneal dialysis solution is either utilized in a single pass or a re-circulation loop. Various re-circulation systems, such as sorbent cartridges or dialyzers, are also known. Work *et al.,* "Catheter" United States Patent 6,749,580 (2004)(herein incorporated by reference).

In another embodiment, the present invention contemplates an improved fixed split-tip dialysis catheter (*i.e.*, for example, HemoSplit^{™}, Bard Access Systems) comprising an anti-adhesion drug combination. Pourchez T., "Multilumen Catheter, Particularly For Hemodialysis" United States Patent No: 6,001,079 (1999)(herein incorporated by reference). Although it is not necessary to understand the mechanism of an invention, it is believed that a fixed split-tip dialysis catheter reduces the risk of lumen damage from the tip being split too far apart during dialysate infusion that can lead to infection and bleeding.

Other dialysis catheters suitable for coatings with compositions described herein are also exemplified by: i) a Uldall Double Lumen Hemodialysis Catheter Tray (Cook Critical Care, Bloomington, IN) - these dialysis catheters are primarily used for vascular access during routine hemodialysis treatment; ii) a Femoral Hemodialysis Set (Cook Critical Care, Bloomington, IN)-these femoral catheters are used for blood withdrawal and infusion; and iii) a Spiral Acute Peritoneal Dialysis Catheter (Cook Critical Care, Bloomington, IN)- these peritoneal catheters have spiral side ports and are used for acute access to the peritoneal cavity and may be percutaneously inserted. A synthetic fiber cuff is affixed to the catheter to allow tissue ingrowth.

One embodiment of the present invention contemplates a composition comprising an anti-adhesion drug combination attached to an *in vivo* blood filter device comprising a dialysis membrane that is implanted within the superior vena cava. In one embodiment, the filter device includes a dialysate cavity which is exposed to the interior surface of the dialysis membrane, with the exterior dialysis membrane surface exposed to the patient's blood within the blood vessel. In another embodiment, the filter device is secured at the end of a multiple lumen catheter through which dialysate fluid is continually directed. Gorsuch R.G., "Apparatus And Method For In Vivo Hemodialysis" United States Patent 6,561,996 (2003)(herein incorporated by reference).

### Vascular Grafts

PTFE vascular grafts are known that have a smooth PTFE luminal surface in an attempt to provide a non-adhesive surface for occlusive blood components. Brauker *et al.,* "Vascular Graft With Improved Flow Surfaces" United States Patent No. 6,517,571 (2003)(herein incorporated by reference). In one embodiment, the present invention contemplates an improved coating for a tubular intraluminal graft comprising a tubular, diametrically adjustable stent having an exterior surface, a luminal surface and a wall having a multiplicity of openings through the wall, and further having a tubular covering of porous expanded PTFE film affixed to the stent, said covering being less than about 0.10 mm thick. Myers D.J., "Intraluminal Stent Graft" United States Patent No: 6,547,815 (2003)(herein incorporated by reference). In one embodiment, the intraluminal graft comprises an improved coating, wherein the coating comprises a drug combination selected from the group including, but not limited to, an antiproliferative, an antiplatelet, an antithrombotic or an anticoagulant.

In an alternative embodiment, the anti-adhesion drug combination coating is contemplated to improve a tubular intraluminal graft comprised of porous expanded PTFE film having a microstructure of nodes interconnected by fibrils, the fibrils being oriented in at least two directions which are substantially perpendicular to each other. Lewis *et al.,* "Tubular Intraluminal Graft And Stent Combination" United States Patent No: 5,993,489 (1999); and Campbell *et al.,* "Thin-Wall Intraluminal Graft" United States Patent No: 6,159,565 (2000)(both herein incorporated by reference). In one embodiment, the graft is bifurcated. Thornton *et al.,* "Kink Resistant Bifurcated Prosthesis" United States Patent No: 6,551,350 (2003)(herein incorporated by reference). In one embodiment, the anti-adhesion drug combination coating is contemplated to improve a thin-wall polyethylene tube. Campbell *et al.,* "Thin-Wall Polytetrafluoroethylene Tube" United States Patent No: 6,027,779 (2000)(herein incorporated by reference). One having skill in the art can realize that a device comprising a polyethylene tube coated with an anti-adhesion drug combination as contemplated herein, is useful to improve any graft or catheter.

### Surgical Material Sheets

In one embodiment, a drug delivery device is placed on the adventitial or periadventitial tissue (*i*.*e*., for example, the outside surface of a blood vessel and/or vascular graft) as a sheet of material. In one embodiment, these combinations are sheets of material as contemplated by the methods and devices described in United States Patent No: 6,534,693 To Fischell et al. (herein incorporated by reference). The methods of the invention are achieved by coating a suitable sheet of material, a mesh, or other suitable matrix on one side or on both sides thereof, or impregnating into such material, mesh, or other suitable matrix with the desired combination of drugs and bringing the combination to the space external to the vascular structure to deliver the desired drugs and achieve the desired effect(s). The matrix can be biodegradable (or bioerodible) or nonbiodegradable (or biostable). The antiproliferative drug and the supplemental or complementary pharmaceutical drug can be mixed together and attached to a delivery device, or such drugs can be attached to a delivery device in discrete layers and/or locations of the device. In one embodiment, the present invention contemplates a composition comprising a sheet of material to which antiproliferative, antiplatelet, antithrombotic or anticoagulant drugs are attached either singly, or in any combination.

The present invention contemplates medical devices to reduce scar tissue and/or adhesion formation following surgical procedures, trauma or wounds. Most surgical procedures require tissue injury wherein the consequential healing process inevitably results in the formation of scar tissue and/or adhesions. Surgical tissue injury may be external or internal and may be performed using an open surgical site or a closed surgical site. The present invention contemplates prevention of scar tissue and/or adhesion formation by administering cytostatic antiproliferative drugs using medical devices both before, during and after surgical procedures that are performed, for example, using a traditional scalpel *(i.e.,* an open surgical site) or using an endoscopic procedure (*i.e.*, a closed surgical site). The present invention also contemplates prevention of fibrin sheaths, scar tissue and/or adhesion formation by administering a GPIIb/IIIa inhibitor. In one embodiment, the antiproliferative drugs are combined with antiplatelet and/or antithrombotic drugs. In another embodiment, the antiproliferative drugs, with or without the antiplatelet and/or antithrombotic drugs, are combined with anticoagulant drugs. In one embodiment, the present invention contemplates a patient having symptoms of end stage renal disease that requires frequent dialysis.

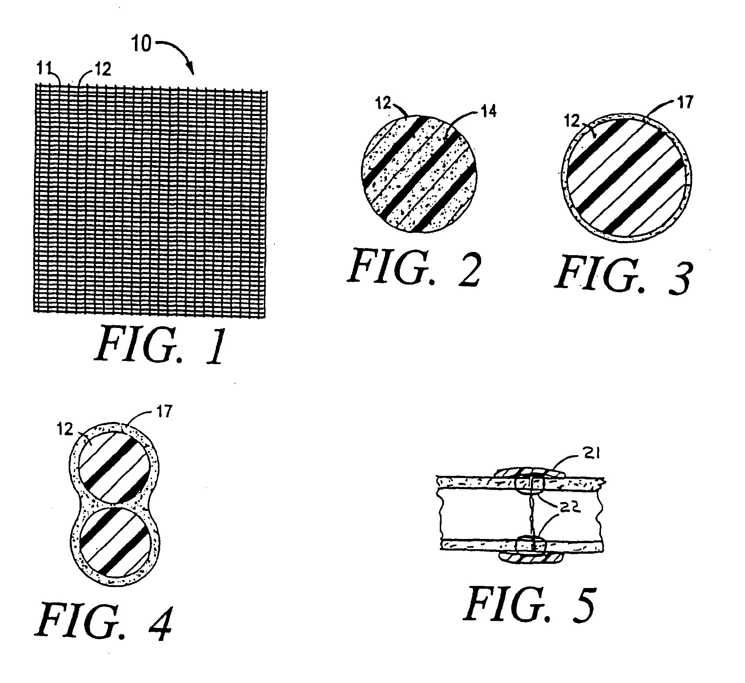

One embodiment of the present invention contemplates a device comprising a surgical material *(i.e.,* for example, a mesh, wrap, sponge, or gauze) wherein a cytostatic antiproliferative drug is attached. Figure 1 shows an absorbable mesh surgical material 10 with mesh strands 12 and open spaces 11. The surgical material 10 is designed to be placed post-operatively into or around biological tissue (*i.e.*, for example, human) at the site of a surgical procedure. When placed at the site of a surgical procedure, the surgical material 10 is designed to slowly elute *(i.e.,* for example, from a controlled release composition) a cytostatic antiproliferative drug so as to decrease the formation of scar tissue and/or adhesions and to reduce the extent of adhesions. When placed generally around biological tissue, the mesh 10 forms a cytostatic antiproliferative surgical wrap. The mesh strands 12 can be made from oxidized regenerated cellulose or other biodegradable materials (*i*.*e*., for example, poly-lactide or poly-glycolide polymers or copolymers) wherein the cytostatic anti-proliferative drug is attached by methods including, but not limited to, being either embedded within the strands, coated onto the outer surfaces of the strands or held onto the strands by adhesion or capillary action. For example, the present invention contemplates one embodiment of a biodegradable polymer composition suitable for making a surgical material in Table 1.

**Table 1: Specifications For A 50/50 D,L, Lactide/Glycolide Co-Polymer**

| **SPECIFICATION** | **VALUE RANGE** |
|---|---|
| Inherent Viscosity | 0.90 dL/g- 1.10 dUg in chloroform at 25°C |
| Copolymer Ratio - Lactide (Mole %) | 45-55 |
| Copolymer Ratio - Glycolide (Mole %) | 45-55 |
| Residual Monomer - Lactide | 0 -1.5 % |
| Residual Monomer - Glycolide | < 0.2 % |
| Residual Solvent | < 0.1 % |
| Appearance | Light tan pellet or granule |
| Pellet Size | Sieved through a 4 mm screen |
| Glass Transition Temperature | 41 - 50°C |
| Sulphated Ash | < 0.02 % |
| Residual Tin | < 100 ppm |
| Moisture | < 2500 ppm |

Figure 2 is an enlargement of a cross section of the mesh of Figure 1 showing a single strand 12 of the mesh 10 in which the cytostatic anti-proliferative drug 14 is attached within the strand 12.

Figure 3 is an enlargement of the cross section of a single strand 12 of Figure 2 where the cytostatic anti-proliferative drug is attached by a coating 17 formed onto the exterior surface of the strand 12. In one embodiment, the strand 12 is formed from either a biostable or biodegradable polymer material. The material of the coating 17 comprises a medium that is selected so that the drug attached to the coating 17 will slowly elute into the biological tissue at the site of a surgical procedure. Preferably, the rate of release of the drug into the adjacent biological tissue may be further adjusted wherein coating 17 is covered with an additional coating (not shown).

Figure 4 is an enlargement of two adjacent strands 12 of the mesh 10 onto which a cytostatic antiproliferative drug 18 is attached. In one embodiment, the cytostatic antiproliferative drug 18 includes, but is not limited to, sirolimus, anti-sense to c-myc (Resten-NG), tacrolimus (FK506), everolimus (SDZ-RAD), CCI-779, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin and 2-desmethyl-rapamycin. Other anti-proliferative drugs may also include cytotoxic cancer drugs such as taxol, actinomycin-D, alkeran, cytoxan, leukeran, cis-platinum, carmustine (BiCNU), adriamycin, doxorubicin, cerubidine, idamycin, mithracin, mutamycin, fluorouracil, methotrexate, thioguanine, taxotere, etoposide, vincristine, irinotecan, hycamptin, matulane, vumon, hexalin, hydroxyurea, gemzar, oncovin and etophophos.

A mesh or surgical material comprising a medium wherein a cytostatic anti-proliferative drug is attached contemplated by the present invention may or may not be biodegradable as long as the mesh or surgical material is biocompatible. In one embodiment, the medium, mesh or surgical material gradually releases the cytostatic anti-proliferative drug into the surrounding surgically injured tissue over a period from as short as a day to as long as a few months, the rate of release being controlled by the type of material into which the drug is placed *(supra).* In one embodiment, a polymer coating is placed over the medium, mesh or surgical material to slow the eluting of the drug into the surrounding tissue. Such polymer materials are known in the field of controlled release formulations. Goldstein *et al.,* "Compositions And Methods For Coating Medical Devices" U.S. Pat. No. 6,143,037 (2000)(herein incorporated by reference). Although it is not necessary to understand the mechanism of a successful invention, it is believed that the effect of the cytostatic anti-proliferative drug attached to at least part of the medium, mesh or surgical material decreases cellular proliferation and therefore decreases the formation of scar tissue and/or adhesions and/or adhesions. Preferably, the mesh 10 wrapped around a vascular anastomosis reduces the narrowing of that vessel which often occurs at the site of an anastomosis.

The'693 patent to Fischell *et al. (supra)* describes various means and methods to reduce scar tissue and/or adhesion formation resulting from a surgical procedure. However, Fischell *et al.* does not describe a cytostatic antiproliferative surgical wrap that is placed around biological tissue of a patient where there is a risk of formation of scar tissue and/or adhesions. Further, Fischell *et. al.* does not describe combining cytostatic antiproliferative drugs *(i.e.,* for example, rapamycin) with either antiplatelet, antithrombotic or anticoagulant drugs. The present invention contemplates various means and methods including, but not limited to, surgical wraps that are placed around a biological vessel organ of a patient where there is a risk of scar tissue, adhesion and thrombus formation. Although several companies have developed products (such as biodegradable mesh, gels, foams and barrier membranes of various materials) that can be placed between these structures to reduce the tissue growth, none are entirely effective. In one embodiment, the present invention contemplates a composition comprising tissue barrier membranes to which antiproliferative, antiplatelet, antithrombotic or anticoagulant drugs are attached either singly, or in any combination.

### Surgical Wraps

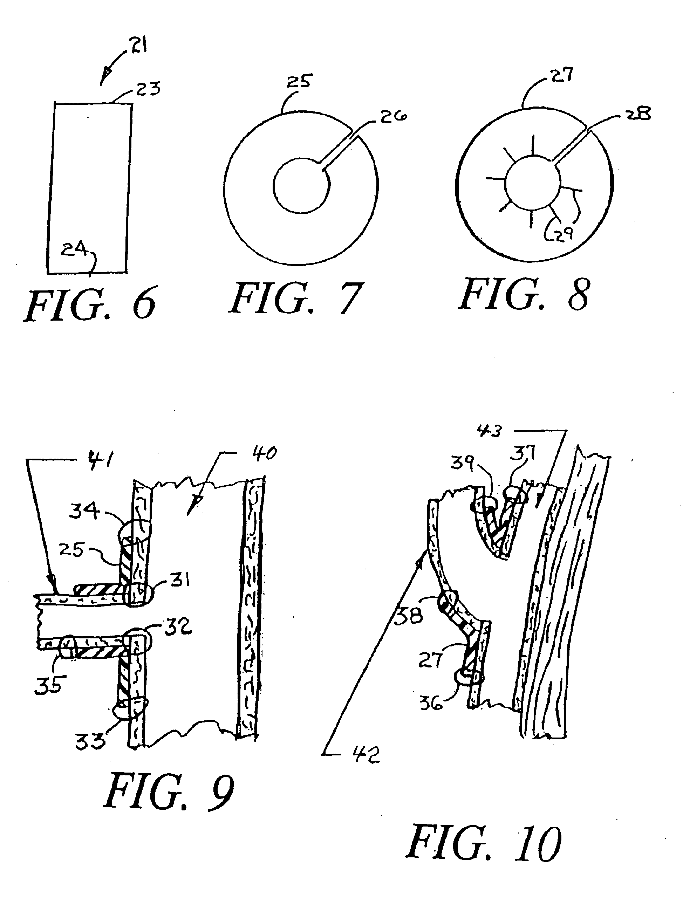

One embodiment of the present invention contemplates a surgical wrap comprising a cytostatic antiproliferative drug *(i.e.,* sirolimus, tacrolimus and analogs of sirolimus) wherein the drug reduces the narrowing of a body vessel, duct or lumen. In one embodiment, the present invention contemplates a composition comprising surgical wrap to which antiproliferative, antiplatelet, antithrombotic or anticoagulant drugs are attached either singly, or in any combination. In one embodiment, the surgical wrap is configured by wrapping to contact the external surface of the vessel, duct or lumen such that as the cytostatic antiproliferative drug is released from the surgical wrap, the drug is absorbed into the surrounding tissue. For example, Figure 5 illustrates a cross section of a cytostatic anti-proliferative surgical wrap 21 shown wrapped around an anastomosis of a vessel, duct or lumen, the sutures 22 being used to join the cut ends of a vessel, duct or lumen. In one embodiment, the surgical wrap may be secured in place with at least one surgical closure such as, but not limited to, a conventional suture or staple and/or sutures or staples to which a cytostatic anti-proliferative drug has been attached. For example, Figure 6 shows such a-surgical wrap 21 having ends 23 and 24, which ends are typically secured to a vessel, duct or lumen that has an anastomosis. The vessel, duct or lumen can include, but is not limited to, a vein, an artery, the joining of an artificial graft to a vein or artery, a ureter, a urethra, a bile duct, an ileum, a jejunum, a duodenum, a colon or a fallopian tube. One having skill in the art should understand that the surgical wrap contemplated by the present invention may be used at any surgical site. For example, the surgical sites contemplated by the present invention include, but are not limited to, the backbone, nerves coming out of a vertebrae, the colon or ileum *etc.*

In one embodiment, the surgical wraps are configured by sliding to contact, or be near to, the external surface of the vessel, duct or lumen such that as the cytostatic antiproliferative drug is released from the surgical wrap, the drug is absorbed into the surrounding tissue. For example, Figure 7 shows an annular surgical wrap 25 having a cut 26, wherein the annular wrap 25 comprises an attached cytostatic anti-proliferative drug (*i*.*e*., for example, sirolimus, tacrolimus and analogs of sirolimus). In one embodiment, a slit annular wrap 27 has a cut 28 and a plurality of slits 29. (See Figure 8) This type of slit annular wrap 27 is particularly well suited, for example, for suturing to an aorta 40 at the site of an anastomosis with the sections between the slits 29 being placed and sutured onto the blood vessel 41 that is joined to the aorta 40. In one embodiment, an annular wrap 25 is configured for a typical anastomosis that occurs during coronary bypass surgery. (See Figure 9) Preferably, a blood vessel 41 (*i*.*e*., for example, a leg vein) is secured to the aorta 40 by sutures 31 and 32. In one embodiment, the annular wrap 25 is secured to the aorta 40 by means of sutures 33 and 34. Alternatively, the annular wrap 25 may be secured to the aorta 40 by staples (not shown), wherein the staples may or may not be bioresorbable.

In the examples described above, both the surgical wrap 21 and the annular wrap 25 would each have attached an anti-proliferative drug as described herein to prevent the formation of scar tissue and/or adhesions when contacting, or being near to, biological tissues including, but not limited to, the blood vessel 41 or aorta 40. The anastomosis exemplified in Figure 9 is a frequent site where the formation of scar tissue and/or adhesions may diminish blood flow by a process known as stenosis. Although it is not necessary to understand the mechanism of a successful invention, it is believed that a controlled release of a cytostatic anti-proliferative drug (*i.e.*, for example, sirolimus, tacrolimus and analogs of sirolimus) from the surgical wrap 21 or the annular wrap 25 reduces the incidence of stenosis at the site of the anastomosis. One of skill in the art should understand, that the above Figures are merely illustrative and that either the surgical wrap 21 or the annular wrap 25 may be used separately, or together, to prevent stenosis following an anastomosis.

In one embodiment, an anastomosis creates a coronary bypass by joining two arteries, wherein the surgical wrap comprising a cytostatic antiproliferative drug is configured to contact the anastomosis site. For example, Figure 10 illustrates a typical coronary artery bypass graft wherein a coronary artery or vein may be joined to a coronary artery. Specifically, Figure 10 depicts an internal mammary artery 42 surgically joined to any coronary artery 43 including, but not limited to, the left anterior descending, left circumflex or right main coronary artery. The administration of a cytostatic antiproliferative drug to decrease the formation of scar tissue and/or adhesions inside the anastomosis is provided by a slit annular wrap 27 that contacts both the coronary artery 43 and the internal mammary artery 42 and is secured by sutures (or staples) 36, 37, 38 and 39. Alternatively, a surgical wrap 21 or an annular wrap 25, either alone or in combination, may also be applied. Furthermore, the surgeon could cut away some of the wrap located between the slits 29 of the slit annular wrap 27 before securing the surgical wrap by sutures or staples to the site of the anastomosis. Although Figure 10 exemplifies an anastomosis joining an internal mammary artery and a coronary artery, any suitable vein could also be used in place of the internal mammary artery.

### Surgical Closures

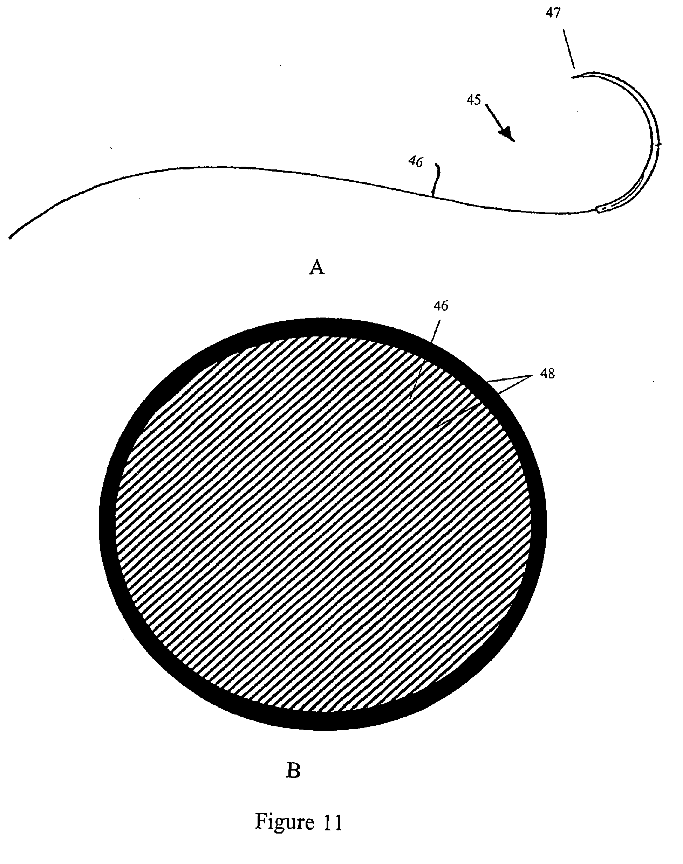

One embodiment of the present invention contemplates a surgical closure *(i.e.,* for example, a suture or a staple) to which a cytostatic antiproliferative drug is attached. Haynes *et al.,* "Drug Releasing Surgical Implant Or Dressing Material" US. Pat. No. 5,660,854 (1997); and Keogh *et al.,* "Method For Attachment Of Biomolecules To Medical Devices Surfaces" U.S. Pat. No. 5,925,552 (1999)(both herein incorporated by reference). In one embodiment, the present invention contemplates a composition comprising surgical closures to which antiproliferative, antiplatelet, antithrombotic or anticoagulant drugs are attached either singly, or in any combination. A drawing of a representative suture 45 and highly enlarged cross section of such a suture comprising an cytostatic antiproliferative drug is shown in Figure 11 A and 11B respectively. Specifically, Figure 11A shows a suture material 46 connected to a needle 47. Further, Figure 11B exemplifies a cross section of suture material 46 which has a cytostatic antiproliferative drug 48 attached (i.e. both external material attachment as well as internal material attachment). In one embodiment, sutures as demonstrated in Figure 11 are used to secure a vascular anastomoses. (See, for example, Figures 9 and 10) Although it is not necessary to understand the mechanism of a successful invention, it is believed that attaching a cytostatic anti-proliferative drug to a suture will reduce scar tissue and/or adhesion formation where the suture penetrates through the biological tissue *(i.e.,* for example, human tissue) therein joining together two vessels, *i.e.,* an anastomosis. In one embodiment, sutures are incorporated at a plurality of locations along the anastomosis.

As with the other embodiments, when desired, the surgical wrap 21, annular wrap 25 or slit annular wrap 27 can be secured in place by a mechanical engagement between each wrap and a vessel, duct or lumen. One securing embodiment of the present invention contemplates the use of transluminally delivered staples which can take on the appearance of rivets. Preferably, these staples are made of an elastomeric material and are bioresorbable.

Surgical closures contemplated by this invention may be either soluble or insoluble. Methods of the present invention contemplate that by using a surgical closure to which a cytostatic anti-proliferative drug is attached, a surgeon can reduce scar tissue and/or adhesion formation on the surface of the skin or anywhere else where surgical closures are used. In one embodiment, placing surgical closures (*i.e*., for example, sutures) contemplated by this invention during eye or plastic surgery will reduce the expected scar tissue and/or adhesion formation which can compromise the result of a surgical procedure. In another embodiment, a cytostatic antiproliferative drug could be attached to any conventional surgical staple that is used to join together human tissue after a surgical procedure. It should also be understood to those skilled in the art that any of the surgical closures contemplated by the present invention *(i.e.,* for example, sutures 22, 31, 32, 33, 34, 35, 36, 37, 38, 39 or 46 as shown in Figures. 5, 9, 10 and 11) could be conventional sutures or could have a cytostatic drug as described herein attached to that closure.

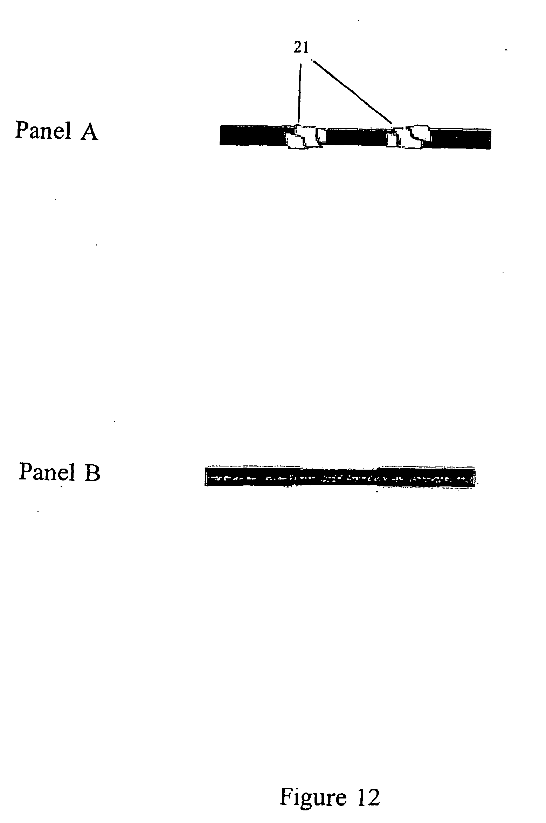

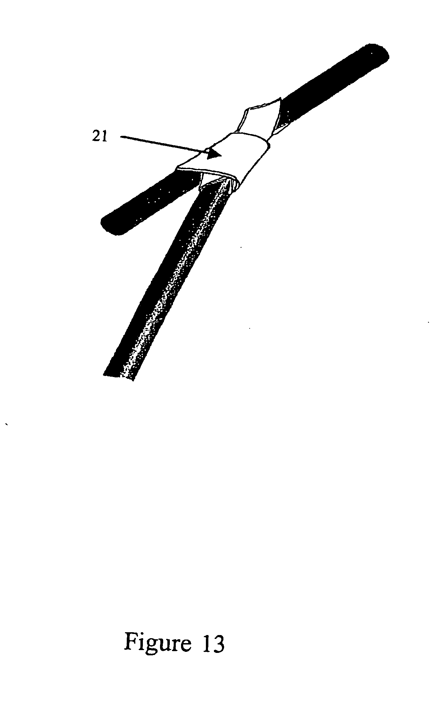

Surgical wraps are especially useful for surgical procedures comprising anastomoses. In one embodiment, an end-to-end arterial anastomosis comprises a surgical wrap 21 placed on the exterior surface of an artery, wherein two anastomoses sites allow the integration of a joining arterial section. The surgical wrap 21 may comprise a cytostatic antiproliferative drug that will reduce subsequent scarring and/or adhesions and vascular stenosis. (See Figure 12, Panel A). After the healing is complete, the arterial anastomosis has reduced scarring and/or adhesions and arterial narrowing. (See Figure 12, Panel B). In another embodiment, an end-to-side anastomosis comprises a surgical wrap 21 placed on the exterior surface of a vein and an artery and/or an arteriovenous graft. The surgical wrap 21 may comprise placement upon both the vein and the artery and/or arteriovenous graft to reduce subsequent scarring and/or adhesions and vascular stenosis. (See Figure 13).

Although it is not necessary to understand the mechanism of a successful invention, it is believed that those skilled in the art would understand that surgical closures including, but not limited to, sutures or staples with a cytostatic anti-proliferative agent attached are useful for joining any biological tissue *(i.e.,* for example, human tissue) resulting in a reduction of cellular proliferation, and consequently, formation of adhesions or scar tissue and/or adhesions.

For example, when cytostatic anti-proliferative sutures are used on the skin's surface, it should be understood that an ointment that includes a cytostatic anti-proliferative drug could be applied to the skin at the site of a surgical incision. Preferably, cytostatic anti-proliferative drugs contemplated the present invention comprise the group including sirolimus, anti-sense to c-myc (Resten-NG), tacrolimus (FK506), everolimus (SDZ-RAD), any other analog of sirolimus including, but not limited to, CCI-779, ABT-578, 7-epi-rapamycin, 7-thiomethyl-rapamycin, 7-epi-trimethoxyphenyl-rapamycin, 7-epi-thiomethyl-rapamycin, 7-demethoxy-rapamycin, 32-demethoxy-rapamycin or 2-desmethyl-rapamycin.

### Adhesions

The present invention contemplates compositions and methods to reduce or inhibit the formation of adhesions. Adhesions is an unsolved medical problem to which current medical practice is largely ineffective.

The rate of adhesion formation after surgery is surprising given the relative lack of knowledge about adhesions within the medical community. Traffic accident victims who undergo surgery form adhesions in 67% of the cases. Weibel et al., Am JSurg. 126:345-353(1973). This number increases to 81% and 93% for patients with major and multiple procedures respectively. Similarly, 93% of patients who had undergone at least one previous abdominal operation had adhesions, compared with only 10.4% of patients who had never had a previous abdominal operation. Menzies et al., Ann R Coll Surg Engl. 72:60-63 (1990). Furthermore, 1 % of all laparotomies develop obstructions due to adhesions within one year of surgery with 3% leading to obstruction at some time after surgery. Similarly, in regards to small bowel obstruction, 60-70% of cases involve adhesions. Following surgical treatment of adhesions causing intestinal obstruction, obstruction due to adhesion reformation occurrs in 11 to 21% of cases. Between 55 and 100% of patients undergoing pelvic reconstructive surgery will form adhesions.

Adhesions are believed to cause pelvic pain by tethering down organs and tissues, causing traction (pulling) of nerves. Nerve endings may become entrapped within a developing adhesion. If the bowel becomes obstructed, distention will cause pain. Some patients in whom chronic pelvic pain has lasted more than six months may develop "Chronic Pelvic Pain Syndrome." In addition to the chronic pain, emotional and behavioral changes appear due to the duration of the pain and its associated stress. According to the International Pelvic Pain Society:
"We have all been taught from infancy to avoid pain. However, when pain is persistent and there seems to be no remedy, it creates tremendous tension. Most of us think of pain as being a symptom of tissue injury. However, in chronic pelvic pain almost always the tissue injury has ceased but the pain continues. This leads to a very important distinction between chronic pelvic pain and episodes of other pain that we might experience during our life: usually pain is a symptom, but in chronic pelvic pain, pain becomes the disease."
Chronic pelvic pain is estimated to affect nearly 15% of women between 18 and 50. Other estimates arrive at between 200,000 and 2 million women in the United States. The economic effects are also quite staggering. It is believed that the direct medical costs for outpatient visits for chronic pelvic pain for the U.S. population of women aged 18-50 years are $881.5 million per year, where 15% report time lost from paid work and 45% report reduced work productivity.

Not all adhesions cause pain, and not all pain is caused by adhesions. In fact, the medical community is not in complete agreement that adhesions cause pain. Adhesions are not easy to observe non-invasively, for example with MRI or CT scans. However, it is clear that a medical relationship exists between pain and adhesions. Of patients reporting chronic pelvic pain, about 40% have adhesions only, and another 17% have endometriosis (with or without adhesions).

### Renal Disease

One embodiment of the present invention contemplates the treatment of patients exhibiting symptoms of a renal disease. In one embodiment, the present invention contemplates treatment of a renal disease comprising a medium to which antiproliferative, antiplatelet, antithrombotic or anticoagulant drugs are attached either singly, or in any combination. Renal diseases may include, but are not limited to, atherosclerosis of the renal artery, atherosclerotic nephropathy, fibromuscular dysplasia and end-stage renal disease.

The optimal treatment of patients with renal diseases is currently in debate. Management options include, but are not limited to, surgical or percutaneous procedures (*i.e.*, for example, angioplasty and stenting). Generally, in patients with fibromuscular disease, the results of surgery and percutaneous approaches are successful. In patients with atherosclerotic diseases, however, the data is less promising.

### Atherosclerotic Renal Artery Stenosis

Atherosclerotic renal stenosis is a rather frequent condition which, because of it's progressive nature, is becoming one of the leading causes of end-stage renal disease. For example, atherosclerotic renal artery stenosis accounts for 12 - 14% of new dialysis patients each year. Atherosclerotic renal artery stenosis may be associated with other clinical disease states including, but not limited to, coronary artery disease, atherosclerotic peripheral vascular disease, malignant hypertension and diabetes mellitus. Morganti et al., "Treatment Of Atherosclerotic Renal Artery Stenosis" J Am Soc Nephrol 13:S 187-S 189 (2002).

The clinical diagnosis of atherosclerotic renal artery stenosis may be determined by noninvasive imaging techniques known in the art. Three distinct clinical syndromes are known associated with atherosclerotic renal artery stenosis: i) renin-dependent hypertension, ii) essential hypertension and iii) ischemic nephropathy. Symptoms associated with atherosclerotic renal artery stenosis include, but are not limited to, abrupt onset or accelerated hypertension, unexplained or chronic azotemia, azotemia induced by angiotensin-converting enzyme inhibitors, asymmetric renal dimensions and congestive heart failure with normal ventricular function. Safian, R.D., "Atherosclerotic Renal Artery Stenosis" Curr Treat Options Cardiovasc Med., 5:91-101 (2003).

Type 2 diabetes mellitus patients may develop atherosclerotic nephropathy that is associated with renal artery stenosis. Subcritical (<65%) renal artery stenosis is known to occur during chronic kidney disease in patients with type 2 diabetes with uncontrolled hypertension and serum creatinine levels of 1.8 mg/dL or greater. The relative risk for progression to end-stage renal disease is greater in those patients having renal stenosis than those without the condition. Myers et al., "Renal Artery Stenosis By Three-Dimensional Magnetic Resonance Angiography In Type 2 Diabetics With Uncontrolled Hypertension And Chronic Renal Insufficiency: Prevalence And Effect On Renal Function" Am J Kidney Dis 41(2):351-359 (2003).

Surgical intervention, such as operative renal artery repair, is a known approach to alleviate symptoms of atherosclerotic renal diseases. Hypertension and parameters associated with renal function (*i*.*e*., for example, estimated glomerular filtration rates, creatinine levels *etc.)* are improved after surgical vascular reconstruction. Cherr et al., "Surgical Management Of Atherosclerotic Renovascular Disease", J Vasc Surg 35:236-245 (2002).

One embodiment of the present invention contemplates a method comprising a patient exhibiting at least one symptom of atherosclerotic renal artery stenosis, wherein a surgical material comprising sirolimus, tacrolimus and analogs of sirolimus is extravascularly placed within the patient during a surgical procedure. In another embodiment, the surgical material may further comprise a drug selected from the group comprising antiplatelet drugs, antithrombotic drugs, or anticoagulant drugs. In one embodiment, at least one symptom of the renal artery stenosis is reduced. In one embodiment, the surgical procedure comprises stenting. In another embodiment, the surgical procedure comprises renovascular reconstruction.

### End-Stage Renal Disease

End-stage renal disease has various causes that requires mechanical removal of water, salt, electrolytes and waste products excreted by normal kidneys or their accumulation will result in death. Removal of these products can be variably achieved by either hemodialysis or peritoneal dialysis. The most common cause of erid-stage renal disease in the US is diabetes mellitus. End-stage renal disease almost always follows chronic kidney failure which has persisted for 10 to 20 years or more. Symptoms of end-stage renal disease may include, but are not limited to, unexplained weight loss, nausea, vomiting, general ill feelings, fatigue, headache, frequent hiccups, generalized itching, little or no urine output, easy bruising or bleeding, bloody vomit or stools, decreased alertness, drowsiness, somnolence, lethargy, confusion, delirium, coma, muscle twitching or cramps, seizures, an increased skin pigmentation (*i.e.*, for example, yellow or brown), nail abnormalities or decreased sensation in the hands, feet, or other areas.

The primary source of morbidity in adult patients subjected to long-term dialysis comprises complications related to vascular access. As with adults, pediatric patients having end-stage renal disease must rely on chronic hemodialysis upon failure of transplantation options. Long-term survival of arteriovenous fistulas and arteriovenous grafts in pediatric patients is even more important in children than adults due to the concomitant increase in treatment duration. Patency rates between arteriovenous fistulas and arteriovenous grafts do not show consistent differences in pediatric patients: one-year - 74% v. 96%; three-year - 59% v. 69%; and five-year - 59% v. 40%. Furthermore, access patency does not correlate with a patient's weight or age at access creation. Access failure due to thrombosis, stenosis and infection occurred more frequently in arteriovenous grafts. Despite these complications, arteriovenous fistulae and arteriovenous grafts are preferable to facilitate long-term hemodialysis treatments. Sheth et al., "Permanent Hemodialysis Vascular Access Survival In Children And Adolescents With End-Stage Renal Disease" Kidney Int 62(5):1864-1869 (2002)

The practical difficulties in maintaining a patent entry for the connection of dialysis tubing has proved to be one of the most significant obstacles to successful long-term treatment. Several other complications that may develop during long-term dialysis of end-stage renal disease patients that include, but are not limited to, vascular calcification, cardiovascular disease, arterial damage, arterial stiffening and vascular stenosis.

### Vascular Access

Chronic hemodialysis requires reliable vascular access. Historically, double lumen catheters introduced into wide bore veins have replaced the traditional Scribner shunt intended as temporary access that reduced complications and morbidity. Cuffed tunneled internal jugular catheters and synthetic arteriovenous grafts usually made of polytetrafluoroethylene (PTFE) improved the vascular access armamentarium, but the arteriovenous fistula remains the life-line for chronic hemodialysis patient. Preferably, however, synthetic arteriovenous grafts are used in elderly and diabetic patients. Arteriovenous synthetic grafts have advantages including, but not limited to, a short maturation time and multiple potential access sites.

Venous stenosis and thrombotic episodes are responsible for approximately 80% of vascular access failures. Vascular access related morbidity accounts for almost 25% of all hospital stays for end-stage renal disease patients and may contribute to as much as 50% of all hospitalization costs. Monitoring and treatment of vascular access failure due to outflow stenosis may be measured by ultrasound dilution and duplex color flow Doppler technique. Conventional and digital subtraction angiography procedures, however, have the additional advantage of visualizing the total vasculature and blood flow. Current treatments to correct vascular access failure due to outflow stenosis include use of percutaneous transluminal angioplasty, stents and surgical correction. The various methods being used to prevent graft stenosis include use of dipyridamole and radiation. Pareek et al., "Angio-Access For Hemodialysis--Current Perspective" J Indian Med Assoc 99(7):382-384 (2001) The present invention contemplates a method to reduce vascular access morbidity and outflow stenosis by administration of a cytostatic antiproliferative drug such as, but not limited to, sirolimus, tacrolimus and analogs of sirolimus.

Patients with hemodialysis vascular access may be evaluated using radiological ultrasound procedures of the peripheral veins of the upper extremities for initial placement of a dialysis fistula and identification of stenosis and thrombosis in misfunctional dialysis fistulas. Preoperative screening enables the identification of a suitable vessel to create a hemodynamically-sound dialysis fistula. Thrombosed fistula and grafts can be declotted by purely mechanical methods or in combination with a lytic drug. Surlan et al., "The Role Of Interventional Radiology In Management Of Patients With End-Stage Renal Disease" Eur J Radiol 46(2):96-114 (2003).

If an arterio-venous fistula shunt is placed into the arm of a dialysis patient, then the same type of cytostatic anti-proliferative agent(s) as described above could be attached to that shunt device to increase the time during which the associated vein in the arm would remain patent. Ideally, the cytostatic anti-proliferative drug could be placed throughout the inner surface of the shunt or it could be placed near the ends where the shunt attaches to the vein or to the artery.

The advent of permanent hemodialysis access has made possible the use of chronic hemodialysis in patients with end-stage renal disease. Although autogenous arteriovenous fistulae remain the conduit of choice, their construction is not always feasible. Consequently, grafts are placed approximately 51% of the time while arteriovenous fistulas are placed only 26% of the time. Prosthetic grafts made of polytetrafluoroethylene (PTFE) are typically the second-line choice for hemoaccess. However, these grafts suffer from decreased rates of patency and an increased number of complications. Anderson et al., "Polytetrafluoroethylene Hemoaccess Site Infections" American Society for Artificial Internal Organs Journal 46(6):S 18-21(2000). In one embodiment, the present invention contemplates the administration of a medium comprising sirolimus, tacrolimus or an analog of sirolimus to a patient having PTFE graft complication. In one embodiment, the medium comprises an antiproliferative, antiplatelet, antithrombotic or anticoagulant drugs, either singly, or in any combination. In one embodiment, the medium is sprayed onto the PTFE graft. In another embodiment, the medium is attached to a surgical wrap that encircles the PTFE graft. In one embodiment, the medium is attached to a surgical sleeve *(i.e.,* a bandage or mesh that is tubular in nature) that is placed or draped onto the exterior surface of the vasculature during the PTFE graft procedure.

### Stenosis

Stenosis, the most common vascular complication, occurs in 1-12% of transplanted renal arteries and represents a potentially curable cause of hypertension following transplantation and/or renal dysfunction. Treatment with percutaneous transluminal renal angioplasty with a stent has been technically successful in 82-92% of the cases, and graft salvage rate has ranged from 80 to 100%. Restenosis, however, occurs in up to 20% of cases. Surlan et al., "The Role Of Interventional Radiology In Management Of Patients With End-Stage Renal Disease" Eur J Radiol 46(2):96-114 (2003).

Central vein stenosis and occlusion is known to occur following upper extremity placement of peripherally inserted central venous catheters and venous ports. Catheter caliber is not believed to affect the development of these central vein abnormalities. Longer durations of catheter dwell times, however, is positively correlated with central vein stenosis or occlusion. In order to preserve vascular access for dialysis fistulae and grafts it is suggested that alternative venous access sites be considered for patients with chronic renal insufficiency and end-stage renal disease. Gonsalves *et al.,* "Incidence Of Central Vein Stenosis And Occlusion Following Upper Extremity PICC And Port Placement" *Cardiovasc Intervent Radiol* 26(1) (2003)

Renal replacement therapy comprises a combination of dialysis and transplantation that is the only means of sustaining life for patients with end-stage renal disease. The present invention contemplates the administration of a cytostatic antiproliferative drug to reduce renal artery stenosis following a kidney transplant. In one embodiment, the reduction in stenosis is due to a diminished presence of atherosclerosis and fibrosis at the anastomosis. Although transplantation is the treatment of choice, the number of donor kidneys are limited and transplants may fail. Hence many patients require long-term or even life-long dialysis. Vale et al., "Continuous Ambulatory Peritoneal Dialysis (CAPD) Versus Hospital Or Home Hemodialysis For End-Stage Renal Disease In Adults" Cochrane Database Syst Rev (1):CD003963 (2003).

In one embodiment, the present invention contemplates a a method to treat stenosis or restenosis comprising a medium comprising an antiproliferative, antiplatelet, antithrombotic or anticoagulant drug, either singly, or in any combination.

### Cardiovascular Complications Of Renal Disease

Cardiovascular complications are known to occur in patients having end-stage renal disease. The present invention contemplates the administration of a complementary pharmaceutical drug comprising a cytostatic antiproliferative drug under conditions such that cardiovascular complications related to end-stage renal disease are reduced. In one embodiment, the present invention contemplates a complementary antiproliferative pharmaceutical drug combination selected from the group comprising an antiplatelet, antithrombotic or anticoagulant drug.

Vascular calcification is common in patients with end-stage renal disease who are treated with regular dialysis, and is known to contribute to the very high mortality rate from cardiovascular causes in such patients. Arterial calcification in those with chronic renal failure can result from the deposition of mineral along the intimal layer of arteries in conjunction with atherosclerotic plaques or from calcium deposition in the medial wall of arteries that is due, at least in part, to disturbances in mineral metabolism. It appears that coronary artery calcification is common and often quite extensive in patients with end-stage renal disease and its appearance may be useful in predicting the risk of adverse cardiovascular events. Goodman W., "Vascular Calcification In End-Stage Renal Disease" J Nephrol 15(Suppl 6):S82-S85 (2002).

Large artery damage is a major contributory factor to the high cardiovascular morbidity of patients with end-stage renal disease. Arterial stiffness (*i*.*e*., for example, carotid distensibility) results from this tissue damage and measurements of this phenomenon may be important to assess cardiovascular risk reduction strategies. Aortic stiffness measurements could serve as an important tool in identifying end-stage renal disease patients having a higher risk of cardiovascular disease. Blacher et al., "Prognostic Significance Of Arterial Stiffness Measurements In End-Stage Renal Disease Patients" Curr Opin Nephrol Hypertens 11(6):629-34 (2002).

For any of the applications described herein, the systemic application of one or more of the cytostatic anti-proliferative agents that have been described could be used conjunctively to further minimize the creation of scar tissue and/or adhesions.

Although only the use of certain cytostatic anti-proliferative agents has been discussed herein, it should be understood that other medications could be added to the cytostatic anti-proliferative drugs to provide an improved outcome for the patients. Specifically, for applications on the skin, an antiseptic, and/or antibiotic, and/or analgesic, and/or antiinflammatory agent could be added to a cytostatic anti-proliferative ointment to prevent infection and/or to decrease pain. These other agents could also be applied for any other use of the cytostatic antiproliferative drugs that are described herein. It is further understood that any human patient in whom a cytostatic antiproliferative agent is used plus at least one of the other drugs listed above could also benefit from the systemic administration of one or more cytostatic anti-proliferative agent that has been listed herein.

Various other modifications, adaptations, and alternative designs are of course possible in light of the above teachings. Therefore, it should be understood at this time that within the scope of the appended claims, the invention can be practiced otherwise than as specifically described herein.

### Experimental

The following is merely intended as a representation of one embodiment of the present invention and is not intended to be limiting.

### Example 1

### Rabbit Pericardial Adhesion Prevention Study

This example predicts the ability of one embodiment of a hydrogel-based bioadhesive comprising sirolimus and analogs of sirolimus, xemilofiban, and bivalirudin to prevent post-surgical adhesions and scarring. Eighteen female New Zeland White Rabbits, 3 - 4 kg in body weight will undergo a standardized pericardial abrasion protocol known in the art. Bennett et al., "Next-Generation HydroGel Films As Tissue Sealants And Adhesion Barriers", J Card Surg 18:1-6 (2003); and Wiseman et al., "Fibrinolytic Drugs Prevent Pericardial Adhesions In The Rabbit" J Surg Res 53:362-368 (1992).

The rabbits will be sedated, placed in dorsal recumbency, intubated, and maintained under inhalation anesthesia. A median sternotomy is performed to expose the heart. The pericardial sac is opened and a standardized superficial abrasion with a dry gauze on the anterior (ventral) surface of the heart will create a "central strip" (CS) of roughened tissue. The rabbits are then randomized into a control group (N = 6) that receives no further treatment, a first treatment group (N = 6) where the hydrogel-based bioadhesive, as contemplated by the present invention, comprises sirolimus and analogs of sirolimus xemilofiban, and bivalirudin and is applied to the abraded anterior epicardium reaching a thickness of approximately 1 - 2 millimeters and a second treatment group (N = 6) where the hydrogel-based bioadhesive is combined with the anticoagulant, heparin, and applied to the abraded anterior epicardium reaching a thickness of approximately 1 -2 millimeters. *In situ* polymerization of the hydrogel occurs thereby generating a film. The tissue is then rinsed four times with 20 ml of buffered isotonic saline. Excess fluids are then suctioned off and the pericardium is left open. The sternum, however, is closed with interrupted sutures and the fascia and skin are closed in layers. During recovery, the rabbits are administered pain medication (*i.e.*, for example, butorphanol 0.1 - 0.2 mg/kg S.C.) at 0, 6 and 12 hours after surgery.

Fourteen days post-surgery the rabbits will be sacrificed and a necropsy performed. A blind scoring protocol determines the extent, tenacity and density of adhesions resulting from the pericardial abrasions.

The results are expected to show that significantly more adhesions are present in the control group when compared to either the first treatment group or the second treatment group. The first treatment group *(i.e.,* treated with a hydrogel-based bioadhesive comprising sirolimus and analogs of sirolimus xemilofiban, and bivalirudin) will show significantly less adhesions than the second treatment group (*i.e.*, treated with hydrogel-based bioadhesive-heparin combination). Adhesion tenacity and density are also expected to decrease in the following order: control > second treatment group > first treatment group.

### Example 2

### General PEA Polymer Materials & Methods

This example presents the basic materials that were used in the following Examples related to PEA efficacy, biocompatibility

### Polymers

Poly(ester amides) (PEA) were manufactured by MediVas, Inc. Poly(D,L-lactide-coglycolides) (PLGA) were purchased from Boehringer-Ingelheim. Poly(n-butyl methacrylate) (PBMA) was purchased from Polysciences.

### Synthesis

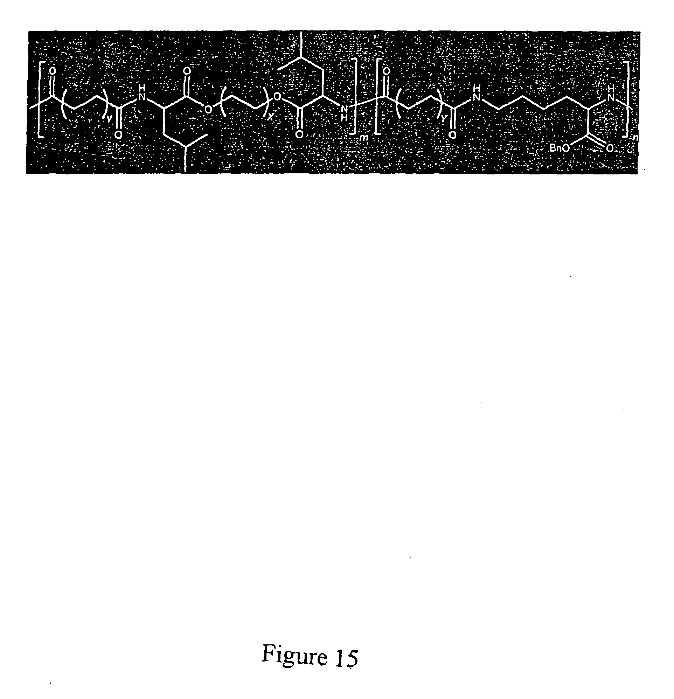

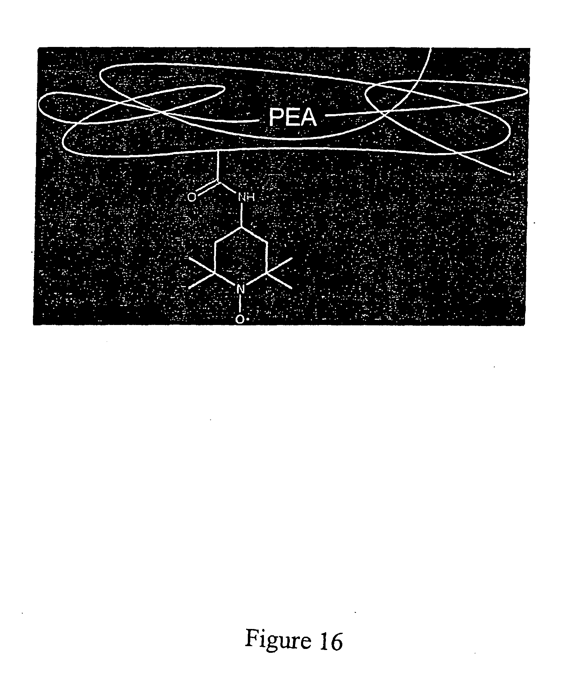

PEA is made in the presence of hexanediol and sebacic acid by synthesizing monomers of two alpha amino acids, L-Leucine and L-Lysine, with diols (x) and diacids (y). See Figure 15. Carboxyl groups of lateral L-Lysine of the polymer chain (BnO) were used as an attachment site to couple drugs or biologics to the polymer backbone. For this study, the nitroxide radical 4-amino TEMPO was conjugated onto PEA. See Figure 16.

### Cell Cultures

Human peripheral blood monocytes were isolated by density centrifugation and magnetic separation (Miltenyi). Human platelets were purchased from the San Diego Blood Bank. Human coronary artery endothelial cells and aortic smooth muscle cells were purchased from Cambrex. Tissue culture polystyrene plates (TCPS; Falcon) with or without fibronectin coating (1 mg/ml) were used as controls.

### Example 3

### Macrophage Development

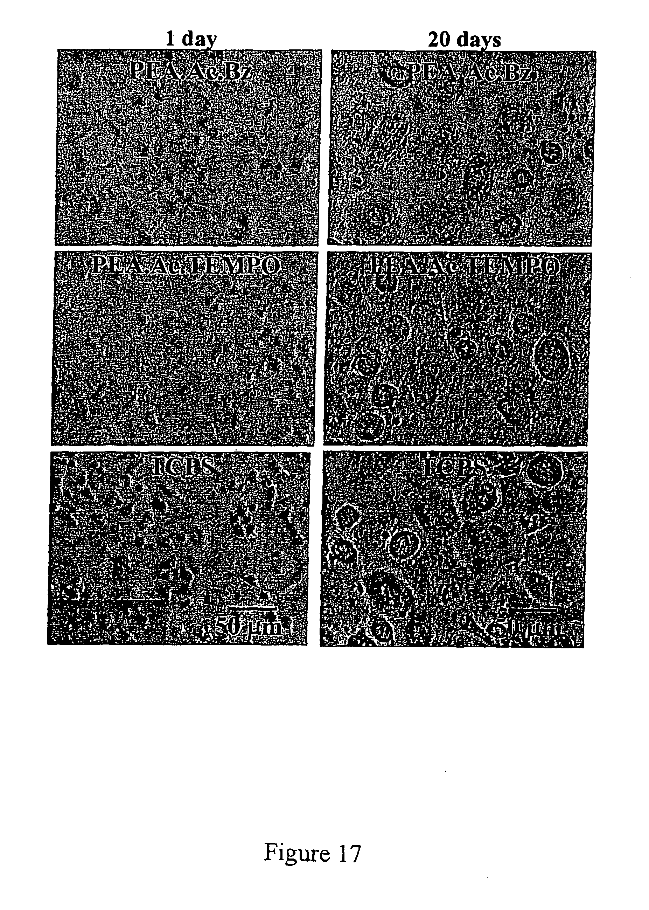

Phenotypic progression of monocytes-to-macrophages and contact-induced fusion to form multinucleated cells proceeded at a similar rate over three weeks of culture. PEA surfaces supported adhesion and differentiation of human monocytes, but, qualitatively, PEA surfaces do not appear to induce a hyper-activated state as judged by morphology and differentiation/fusion rates. See Figure 17.

### Example 4

### Monocyte/Macrophage Activation

Secretion of pro-inflammatory and anti-inflammatory mediators by monocytes and macrophages were measured by ELISA (R&D Systems) after 24 hours (shown) and 7 days (not shown) of incubation on the polymers.

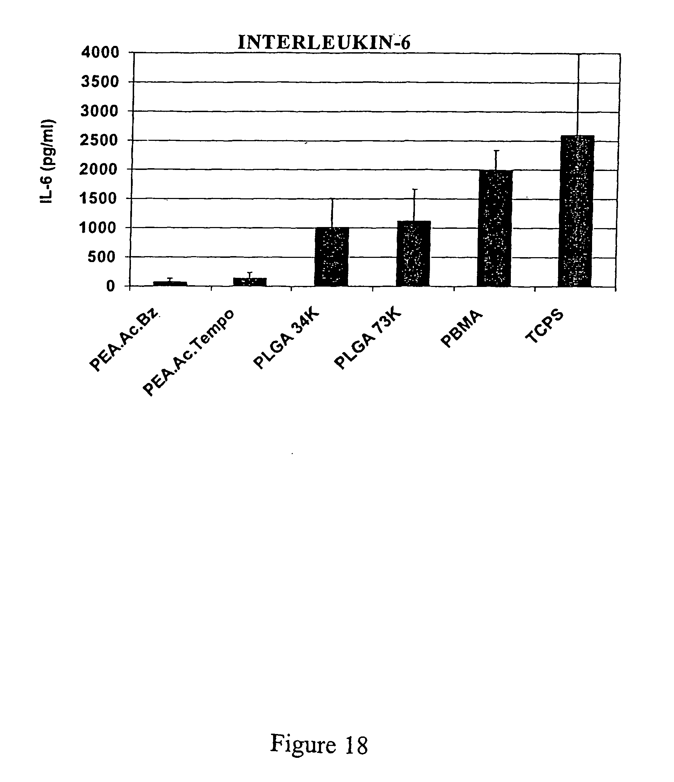

Interleukin-6 is a pleiotropic pro-inflammatory cytokine that can increase macrophage cytotoxic activities. Monocytes secreted over 5-fold less IL-6 when on PEAs than on the other polymers (representative experiment of n=4). IL-6 secretion was less than 10 pg/ml on all polymers by day 7 of culture (not shown). See Figure 18.

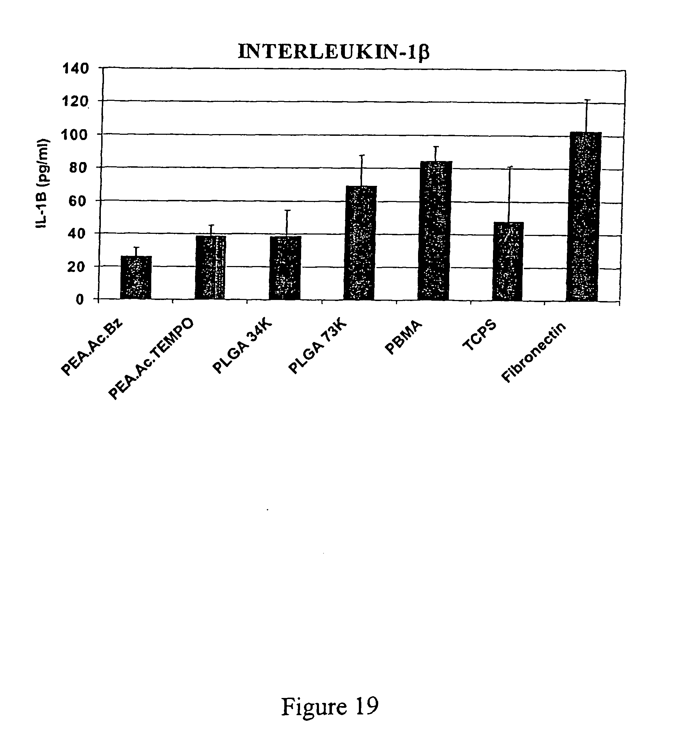

InterIeukin-1 b is a potent pro-inflammatory cytokine that can increase the surface thrombogenicity of the endothelium. After 24 hours, monocytes on PEA secreted less IL-1b than on PLGA 73K and PBMA (representative experiment of n=4). IL-1b secretion was less than 10 pg/ml on all polymers by day 7 of culture (not shown). See Figure 19.

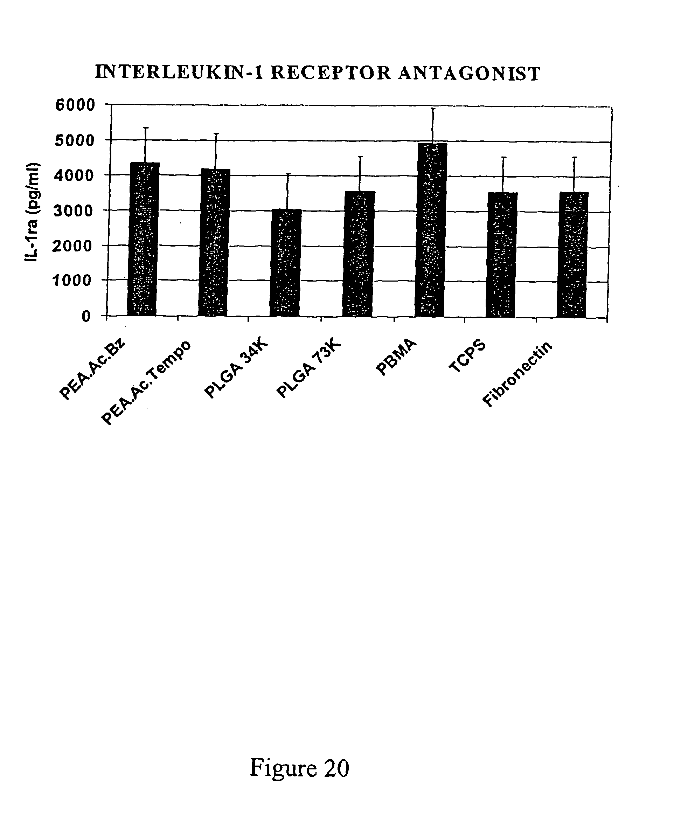

Interleukin-1 receptor antagonist is a naturally occurring inhibitor of IL-1 b that competitively binds the receptors for IL-1 and block pro-inflammatory signaling. PEAs induced adherent monocytes to secrete a significant amount of this anti-inflammatory mediator (representative experiment of n=4). See Figure 20.

### Example 5

### Platelets

### Adhesion And Aggregation

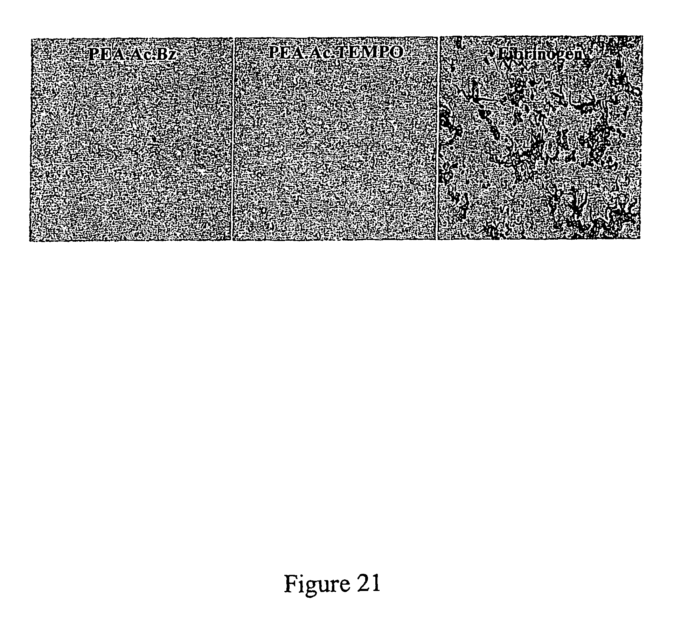

Platelet aggregation, used as a marker of polymer hemocompatibility, was visualized using human platelets that were exposed to PEA and a fibrinogen-coated surface for 30 minutes. Platelets did not readily adhere to or aggregate on PEA but aggregated as expected on fibrinogen. See Figure 21.

### Activation

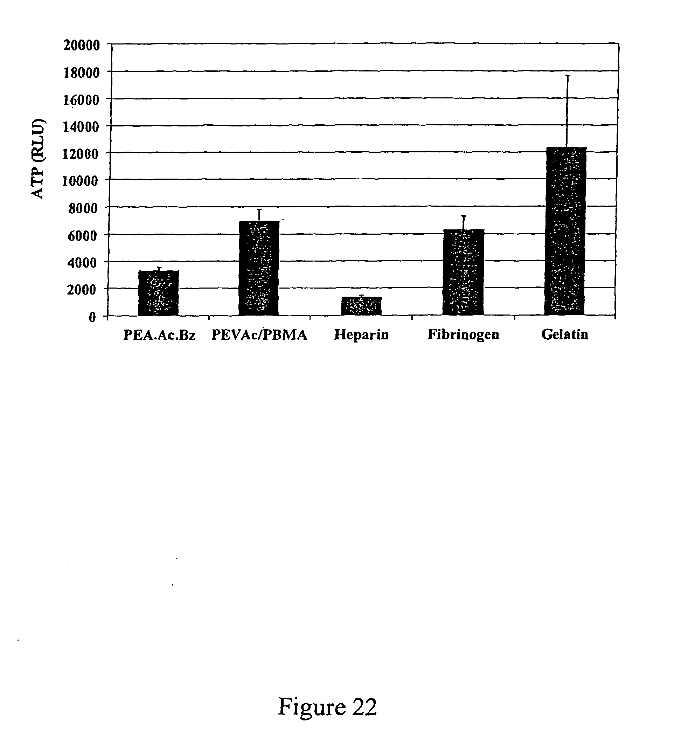

Human platelets were incubated with polymer-coated or protein-coated wells for 30 minutes at 37°C, and ATP release was measured by luminescence assay (Cambrex). Platelets were 2-fold less activated on PEA than on PEV Ac/PBMA, and PEA was only about 2 times as activating to platelets as a heparin-coated surface, suggesting that PEA is highly hemocompatible. See Figure 22.

### Example 6

### Endothelial Cell Biocompatibility

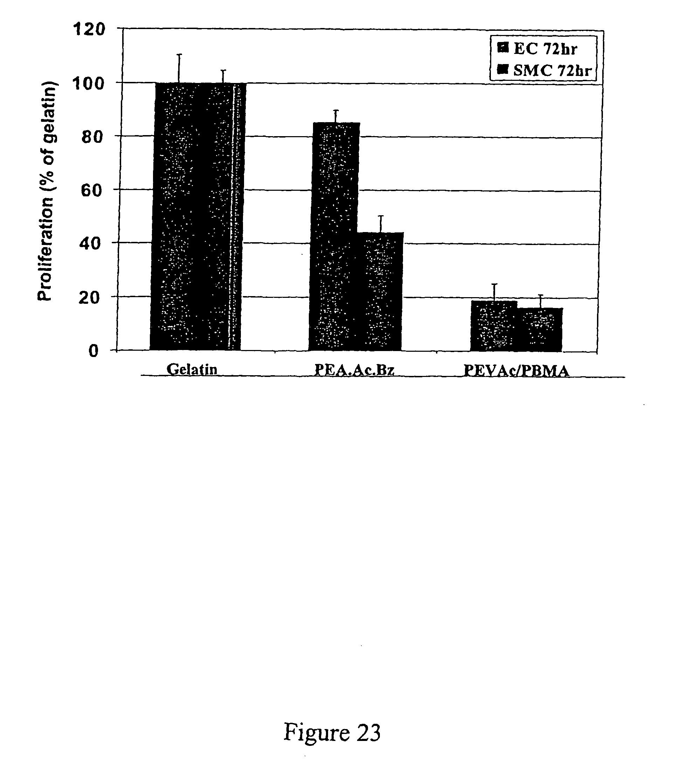

Human coronary artery endothelial cells (EC) and aortic smooth muscle cells (SMC) were incubated on the polymers for 72 hours. EC proliferation on PEA was 4-fold higher than on PEVAc/PBMA, and PEA supported EC growth more than SMC growth relative to the cells' growth on a gelatin-coated surface. See Figure 23.

### Example 7

### Enzyme Biodegradation

Polymers were cast onto stainless steel disks and were incubated in enzyme or control buffers at 37°C. Solutions were changed every 48 hours, and the activity of the enzyme, α-chymotrypsin (CT) was confirmed by a fluorescent substrate assay. At the indicated time points, polymer samples were rinsed and dried to a constant weight. Weight loss was measured gravimetrically, and molecular weight was measured using gel permeation chromatography (GPC).

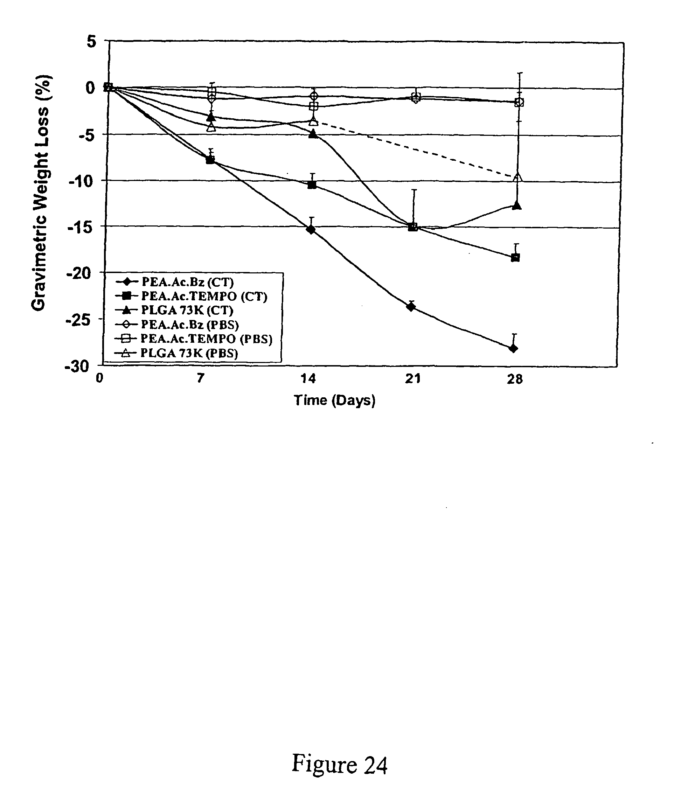

Both PEAs degraded enzymatically with significant weight loss over one month compared to no weight loss in a saline solution (PBS). PLGA, which is known to degrade via hydrolytic bulk erosion, does not degrade enzymatically as shown by the early time points. The samples eventually lose physical integrity, which increases the measurement error. No weight loss was observed for PBMA in buffer with or without enzyme (not shown). See Figure 24.

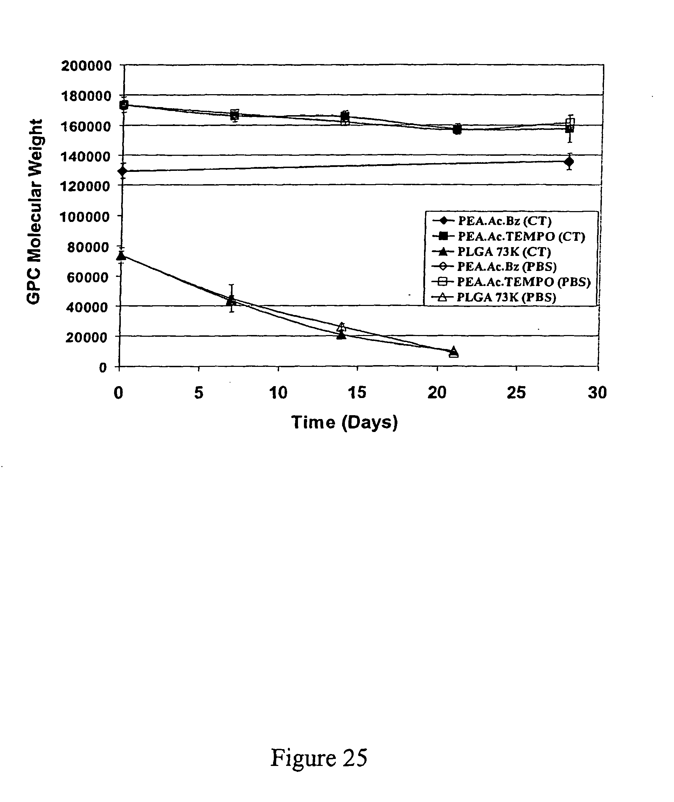

No significant MW change was observed for the PEAs in both PBS and chymotrypsin solutions. A significant MW change (87% decrease at 21 day) was observed for PLGA in both PBS and chymotrypsin solutions. At day 28, the MW of PLGA was too small to be determined by GPC. See Figure 25.

Taken together, these results support an enzymatically-driven, surface erosion mechanism for PEAs compared to the known bulk erosion of PLGAs.

## Claims

1. A method of preventing, inhibiting or reducing fibrin sheath, scar tissue, or adhesion formation, comprising:
administering a GPIIb/IIIa inhibitor to a patient to prevent, inhibit or reduce fibrin sheath, scar tissue, or adhesion formation.

2. The method of claim 1, wherein the GPIIb/IIIa inhibitor is administered to a patient undergoing or following a surgical procedure.

3. The method of claim 1, wherein the GPIIb/IIIa inhibitor is selected from the group consisting of xemilofiban, cromafiban, elarofiban, orbofiban, roxifiban, sibrafiban, RPR 109891, UR-4033, UR-3216, UR-2922, abciximab, tirofiban, and eptifibatide.

4. The method of claim 1, wherein the GPIIb/IIIa inhibitor is selected from the group consisting of xemilofiban, abciximab, tirofiban, and eptifibatide.

5. The method of claim 2, wherein the surgical procedure results in, or at is at risk for developing, fibrin sheath, scar tissue or adhesion formation.

6. The method of claim 2, wherein the surgical procedure is selected from the group consisting of a kidney transplant, an anastomosis, and a dialysis catheter placement procedure.

7. The method of claim 1 further comprising:
a) providing;
i) a patient, and
ii) a composition comprising the GPIIb/IIIa inhibitor, wherein
the composition is administered to the patient to prevent, reduce or inhibit scar tissue or adhesion formation.

8. The method of claim 7, wherein the composition comprises a liquid or a gel.

9. The method of claim 8, wherein the composition is administered to the patient as a liquid spray.

10. The method of claim 1 further comprising:
a) providing;
i) a patient, and
ii) a composition comprising the GPIIb/IIIa inhibitor, wherein
the composition is administered to the patient to prevent, reduce or inhibit fibrin sheath formation.

11. The method of claim 1 further comprising administering an antithrombin drug.

12. The method of claim 11, wherein the steps of administering comprise administering a composition in which the composition comprises the GPIIb/IIIa inhibitor and the antithrombin drug.

13. The method of claim 1 further comprising administering an anticoagulant drug.

14. The method of claim 13, wherein the steps of administering comprise administering a composition in which the composition comprises the GPIIb/IIIa inhibitor and the anticoagulant drug.

15. The method of claim 13, wherein the anticoagulant drug comprises a Factor Xa inhibitor or a Factor VIIa inhibitor.

16. The method of claim 1, wherein the step of administering comprises administering a composition in which the composition comprises the GPIIb/IIIa inhibitor and a polymer-based medium.

17. The method of claim 16, wherein the polymer-based medium provides a controlled release elution of the GPIIb/IIIa inhibitor.

18. The method of claim 16, wherein the polymer-based medium comprises a PEA polymer.

19. The method of claim 16, wherein the polymer-based medium comprises lysine or leucine or combinations thereof.

20. The method of claim 16, wherein the polymer-based medium comprises poly(amino acids).

21. The method of claim 16, wherein the polymer-based medium comprises polyethylene glycol.

22. The method of claim 16, wherein the polymer-based medium comprises a biodegradable polymer.

23. The method of claim 1, wherein the step of administering comprises administering the GPIIb/IIIa inhibitor via a catheter, a graft, a membrane barrier, a wrap or surgical material.

24. The method of claim 23 wherein the catheter, graft, membrane barrier, wrap or surgical material comprises the GPIIb/IIIa inhibitor.

25. The method of claim 24, wherein the step of administering the GPIIb/IIIa inhibitor is via a dialysis/apheresis catheter, a dialysis catheter, a peritoneal dialysis catheter, a fixed-tip dialysis catheter, a peritoneal catheter, a femoral catheter, a synthetic vascular graft, an antiadhesion membrane barrier, a vascular wrap, or a surgical material.

26. The method of claim 24, wherein the surgical material is a surgical mesh or a surgical wrap.

27. The method of claim 24, wherein the surgical material is an annular surgical wrap or a slit annular surgical wrap.

28. A method comprising:
a) providing;
i) a patient, and
ii) a medical device comprising a composition, in which the composition comprises a GPIIb/IIIa inhibitor; and
b) contacting the medical device to the patient to prevent, inhibit or reduce fibrin sheath, scar tissue, or adhesion formation.

29. The method of claim 28, wherein the composition is administered to a patient undergoing or following a surgical procedure.

30. The method of claim 28, wherein the GPIIb/IIIa inhibitor is selected from the group consisting of xemilofiban, cromafiban, elarofiban, orbofiban, roxifiban, sibrafiban, RPR 109891, UR-4033, UR-3216, UR-2922, abciximab, tirofiban, and eptifibatide.

31. The method of claim 28, wherein the GPIIb/IIIa inhibitor is selected from the group consisting of xemilofiban, abciximab, tirofiban, and eptifibatide.

32. The method of claim 29, wherein the surgical procedure results in, or is at risk for developing, fibrin sheath, scar tissue, or adhesion formation.

33. The method of claim 29, wherein the surgical procedure is selected from the group consisting of a kidney transplant, an anastomosis, and a dialysis catheter placement procedure.

34. The method of claim 28, wherein contacting the medical device to the patient is to prevent, inhibit or reduce scar tissue or adhesion formation.

35. The method of claim 28, wherein contacting the medical device to the patient is to prevent, inhibit or reduce fibrin sheath formation.

36. The method of claim 28 further comprising administering an antithrombin drug.

37. The method of Claim 36, wherein the composition comprises the GPIIb/IIIa inhibitor and the antithrombin drug.

38. The method of claim 28 further comprising administering an anticoagulant drug.

39. The method of claim 38, wherein the composition comprises the GPIIb/IIIa inhibitor and the anticoagulant drug.

40. The method of claim 38, wherein the anticoagulant drug comprises a Factor Xa inhibitor or a Factor VIIa inhibitor.

41. The method of claim 28, wherein the composition further comprises a polymer-based medium.

42. The method of claim 41, wherein the polymer-based medium provides controlled release elution of GPIIb/IIIa inhibitor.

43. The method of claim 41, wherein the polymer-based medium comprises a PEA polymer.

44. The method of claim 41, wherein the polymer-based medium comprises lysine, leucine, or combinations thereof.

45. The method of claim 41, wherein the polymer-based medium comprises poly(amino acids).

46. The method of claim 41, wherein the polymer-based medium comprises polyethylene glycol.

47. The method of claim 41, wherein the polymer-based medium comprises a biodegradable polymer.

48. The method of claim 28, wherein the medical device comprises a catheter, a graft, a membrane barrier, a wrap, or a surgical material.

49. The method of claim 48, wherein the medical device comprises a dialysis/apheresis catheter, a dialysis catheter, a peritoneal dialysis catheter, a fixed-tip dialysis catheter, a peritoneal catheter, a femoral catheter, a synthetic vascular graft, an antiadhesion membrane barrier, a vascular wrap, or a surgical material.

50. The method of claim 49, wherein the surgical material is a surgical mesh or a surgical wrap.

51. The method of claim 49, wherein the surgical material is an annular surgical wrap or a slit annular surgical wrap.
